(19) Europäisches Patentamt
European Patent Office
Office européen des brevets

(11) **EP 3 816 297 A1**

(12) **EUROPÄISCHE PATENTANMELDUNG**

(43) Veröffentlichungstag:
**05.05.2021 Patentblatt 2021/18**

(51) Int Cl.:
***C12P 17/04*** *(2006.01)*

(21) Anmeldenummer: **20188856.7**

(22) Anmeldetag: **20.02.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.02.2016 EP 16156410**

(62) Dokumentnummer(n) der früheren Anmeldung(en) nach Art. 76 EPÜ:
**17706448.2 / 3 417 067**

(71) Anmelder: **BASF SE**
**67056 Ludwigshafen am Rhein (DE)**

(72) Erfinder:
- **BREUER, Michael**
  **64285 Darmstadt (DE)**
- **SIEGEL, Wolfgang**
  **67117 Limburgerhof (DE)**
- **RÜDENAUER, Stefan**
  **69469 Weinheim (DE)**
- **PELZER, Ralf**
  **37699 Fürstenberg (DE)**

(74) Vertreter: **Reitstötter Kinzebach**
**Patentanwälte**
**Sternwartstrasse 4**
**81679 München (DE)**

Bemerkungen:
•Das gesamte Dokument mit Referenztabelle(n) und Sequenzliste(n) kann von der Webseite des EPA heruntergeladen werden
•Diese Anmeldung ist am 31-07-2020 als Teilanmeldung zu der unter INID-Code 62 erwähnten Anmeldung eingereicht worden.

# (54) BIOKATALYTISCHE ZYKLISIERUNG VON MEHRFACH UNGESÄTTIGTEN CARBONSÄUREN-VERBINDUNGEN

(57) Die vorliegende Erfindung betrifft Verfahren zur Herstellung von Sclareolid und verwandten Verbindungen durch biokatalytische Zyklisierung einer mehrfach ungesättigten Carbonsäureverbindungen, insbesondere von Homofarnesylsäure und verwandten Verbindungen; ein sowie ein Verfahren zur Herstellung von Ambroxid über die biokatalytische Cyclisierung von Homofarnesylsäure zu Sclareolid.

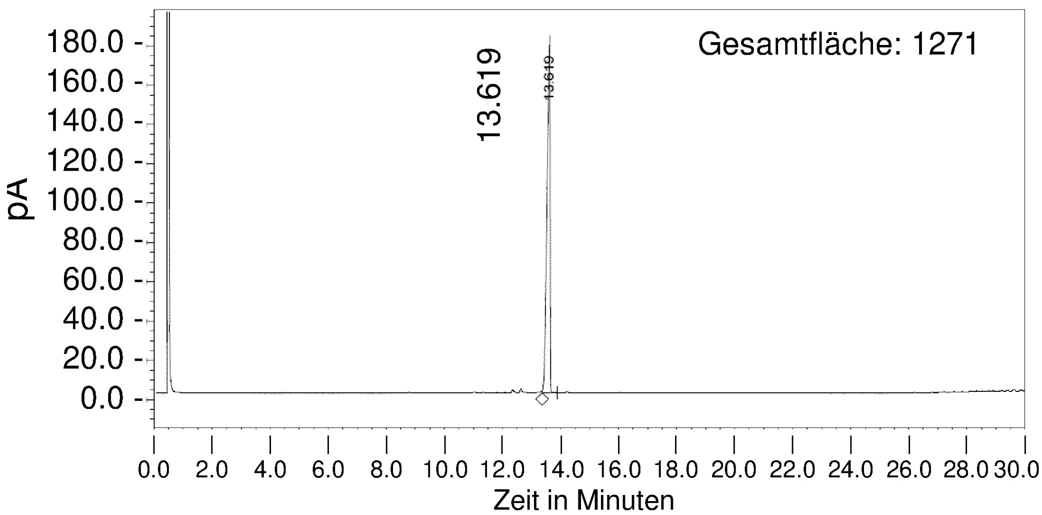


Fig. 1A

EP 3 816 297 A1

Printed by Jouve, 75001 PARIS (FR)

## Beschreibung

Die vorliegende Erfindung betrifft Verfahren zur Herstellung von Sclareolid und verwandten Verbindungen durch biokatalytische Zyklisierung einer mehrfach ungesättigten Carbonsäureverbindungen, insbesondere von Homofarnesylsäure und verwandten Verbindungen; ein sowie ein Verfahren zur Herstellung von Ambroxid über die biokatalytische Cyclisierung von Homofarnesylsäure zu Sclareolid.

## Hintergrund der Erfindung

Verbindungen mit dem Grundgerüst des Dodecahydronaphtho[2,1-b]furans sind als Aromachemikalien von großem wirtschaftlichem Interesse. Hier ist Verbindung **(-)-2** zu nennen, das als das linksdrehende Stereoisomer des Ambroxans bekannte (3aR,5aS,9aS,9bR)-3a,6,6,9a-Tetramethyldodecahydronaphtho-[2,1-*b*]-furan.

Ursprünglich aus dem Ambra des Pottwals gewonnen, gibt es heute vor allem zwei Routen, die Zugang zu Ambroxan erlauben. Sclareol **(3),** ein Inhaltsstoff des Muskatellersalbeis (*Salvia sclarea*) dient dabei oft als geeignetes Ausgangsmaterial für semisynthetisches Material, weil es die optische Information für Verbindung (**(-)-2**) bereits enthält. Dabei kann man den oxidativen Abbau mit Chromsäure, Permanganat, $H_2O_2$ oder Ozon durchführen [Stoll et al.; Helv Chim Acta (1950), 33: 1251]. Das erhaltene Sclareolid **(4)** wird durch eine anschließende Reduktion (z.B. mit $LiAlH_4$ oder $NaBH_4$) in das Ambrox-1,4-diol **(5)** überführt [Mookherjee et al.; Perfumer and Flavourist (1990), 15: 27]. Die Herstellung von Verbindung **(4)** aus Sclareol **(3)** kann auch durch eine Biotransformation mit *Hyphozyma roseoniger* erfolgen [EP 204009]

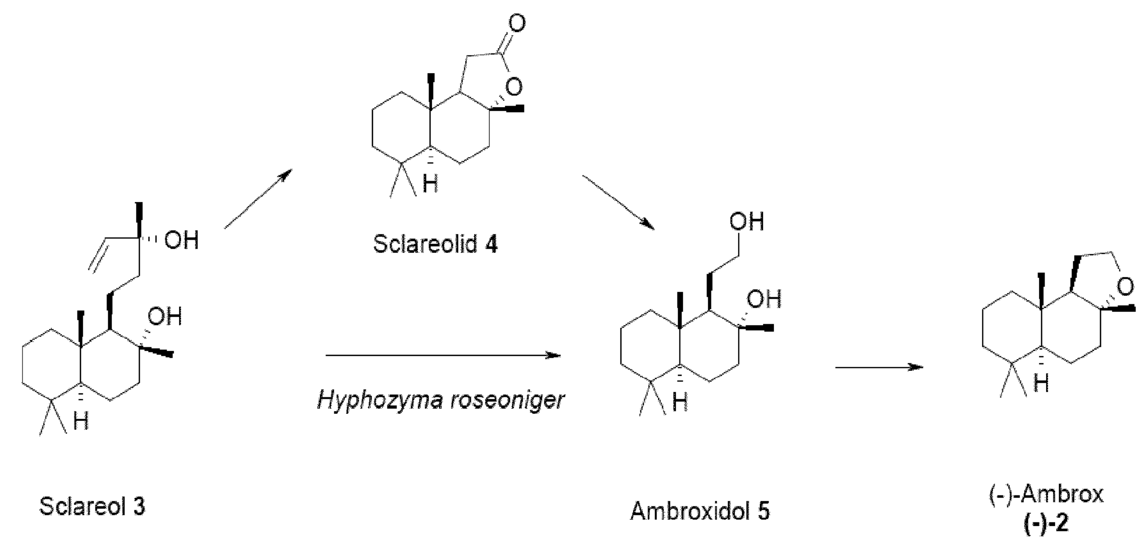

Sclareolid 4
Sclareol 3
Ambroxidol 5
(-)-Ambrox
**(-)-2**

Ambrox-1,4-diol **(5)** kann schließlich mit einer Reihe von chemischen Verfahren zu Verbindung ((-)-**2**) zyklisiert werden. Zu dem Racemat von Ambroxan (*rac*-**2**) sind Zugänge u.a. *via* Homofarnesylsäure [US 513,270; Lucius et al.; Chem Ber (1960), 93: 2663] und 4-(2,6,6-Trimethyl-cyclohex-1-enyl)-butan-2-on [Büchi et al.; Helv Chim Acta (1989), 72: 996] bearbeitet worden.

Das Marktvolumen von Ambroxan lag 2002 mittelwertig bei 20 Tonnen pro Jahr. Hierfür ist eine Ausgangsbasis von ca. 33 Tonnen Sclareol pro Jahr notwendig. Für die Produktion einer Tonne Ambroxan werden 207 Tonnen verschiedener Einzelstoffe benötigt, die wiederum den Anfall von 206 Tonnen Abfall bedingen. Die anfallenden Substanzen weisen unterschiedliche, insgesamt aber verhältnismäßig starke Umwelt- und Gesundheitswirkungen auf [Deutsche Bundesstiftung Umwelt]. Somit ist diese Synthese sehr energieaufwändig und erfordert den Einsatz umweltbelastender Chemikalien.

Die biokatalytische Synthese von Verbindung ((-)-**2**) ist in der Literatur beschrieben [Neumann et al.; Biol Chem Hoppe Seyler (1986), 367: 723]. Dabei wird das Molekül aus Homofarnesol (Verbindung (**1a**), (3*Z*,7*E*)-4,8,12-Trimethyltrideca-3,7,11-trien-1-ol) gewonnen. Als Katalysator wurde die Squalen-Hopen-Zyklase (SHC) aus *Alicyclobacillus acidocaldarius* (ehem. *Bacillus acidocaldarius*) verwendet. Weitere Enzyme die Zyklisierung von Homofarnesol zu Ambroxan katalysieren sind in Patentschriften (z.B. WO 2012/066059) und in der Literatur beschrieben [Neumann et al, a.a.O].

Seitz, M. et al beschreiben in ChemBioChem 2013, 14, 436-439 ein enzymatisches Verfahren zur Herstellung von Sclareolid aus Homofarnesylsäure unter Verwendung der Squalen-Hopen Cyclase aus *Zymomonas mobilis* (*Zm*

SHCl). Die dabei erzielten Produktausbeuten liegen jedoch nur bei etwa 7,7 bis 22,9 %.

Aufgabe der vorliegenden Erfindung war es, ein verbessertes Verfahren zur Herstellung von Ambroxid-Vorstufen insbesondere von Sclareolid und verwandten Verbindungen zur Verfügung zu stellen, das technisch einfacher und kostengünstiger als herkömmliche chemische Verfahren (z.B Verringerung der Anzahl der notwendigen Reaktionsstufen, und/oder günstigere Ausgangsstoffe) durchgeführt werden kann. Eine weitere Aufgabe war es, die anfallenden Kosten zusätzlich durch die Verwendung leicht zugänglicher Ausgangsstoffe sowie einer Verminderung der Anzahl chemischer Reaktionen (bzw. Stufen) zu senken. Insbesondere sollte ein verbessertes biokatalytisches Verfahren zur Herstellung von Sclareolid bereitgestellt werden.

## Kurzfassung der Erfindung

Obige Aufgaben wurde gelöst durch Bereitstellung eines Verfahrens zur Herstellung von Ambroxid-Vorstufen bevorzugt Sclareolid, der allgemeinen Formel **(4)** dadurch gekennzeichnet, dass Homofarnesylsäure-Derivate, insbesondere Homofarnesylsäure der allgemeinen Formel (Fehler! Verweisquelle konnte nicht gefunden werden.) biokatalytisch, wie im folgenden näher erläutert, zyklisiert wird.

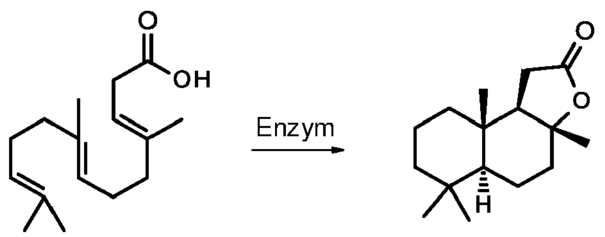


**1b** **4**

## Figurenbeschreibung

Figur 1 zeigt das Gaschromatographie (GC) Spektrum (A) eines erfindungsgemäß hergestellten Zyklisierungsprodukts von Homofarnesylsäure im Vergleich zum (B) dem GC eines käuflichen Sclareolid-Präparats.

## Detaillierte Beschreibung der Erfindung

### A. Allgemeine Definitionen

"Homofarnesylsäure", (Verbindung (**1b**)) ist gleichbedeutend mit, "(3*E*,7*E*)-4,8,12-trimethyltrideca-3,7,11-triensäure "

"Sclareolid" (Verbindung **(4))** ist gleichbedeutend mit "(3aR,5aS,9aS,9bR)-3a,6,6,9a-tetramethyl-1,4,5,5a,7,8,9,9b-octahydrobenzo[e]benzofuran-2-on".

Linksdrehendes Sclareolid (oder Verbindung **(-)-4)** besitzt folgende Formel

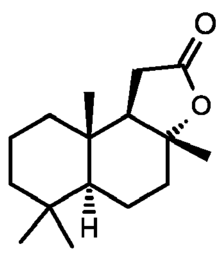

"Ambrox", "Ambroxan" und "Amroxid" werden hierin als Synonyme verwendet. Sie umfassen sämtliche stereoisomeren Formen, wie insbesondere (+)-Ambrox, 3a-epi-(-)Ambrox, 9b-epi-(-) Ambrox und insbesondere (-) Ambrox.

"Cyclasen" im Sinne der vorliegenden Erfindung sind allgemein Enzyme bzw. Enzymmutanten, welche insbesondere die Aktivität einer Homofarnesylsäure-Cyclase zeigen. Als Enzyme mit der Aktivität einer Homofarnesylsäure-Cyclase sind intramolekulare Transferasen aus der Subklasse der Isomerasen; also Proteine mit der EC-Nummer EC 5.4 geeignet. (Enzymcode gemäß Eur. J. Biochem. 1999, 264, 610-650) Insbesondere handelt es sich um Vertreter von EC 5.4.99.17. Als Enzyme mit der Aktivität einer Homofarnesylsäure-Cyclase sind insbesondere solche Cyclasen geeignet, die auch die Cyclisierung von Homofarnesylsäure zu Sclareolid und/oder von Squalen zu Hopen (daher auch

zuweilen die Bezeichnung "SHC" Squalen Hopen Cyclase) bewirken und die ausführlich in der internationalen Anmeldung PCT/EP2010/057696 beschrieben werden, worauf hier ausdrücklich Bezug genommen wird. Mutantan davon sind z.B.beschrieben in der WO 2012/066059 worauf hier ausdrücklich Bezug genommen wird.

Aufgrund der Reversibilität enzymatischer Reaktionen betrifft die vorliegende Erfindung die hierin beschriebenen enzymatischen Umsetzungen in beiden Umsetzungsrichtungen.

"Funktionale Mutanten" einer "Cyclase" umfassen die unten definierten "funktionalen Äquivalente" solcher Enzyme.

Der Begriff "biokatalytisches Verfahren" betrifft jegliches in Gegenwart von katalytischer Aktivität einer erfindungsgemäßen "Cyclase" bzw. eines Enzyms mit "Cyclase Aktivität" durchgeführtes Verfahren, d.h. Verfahren in Gegenwart von rohem, oder gereinigtem, gelöstem, dispergiertem oder immobilisiertem Enzym, oder in Gegenwart ganzer mikrobieller Zellen, welche derartige Enzymaktivität aufweisen oder exprimieren. Biokatalytische Verfahren umfassen somit enzymatische als auch mikrobielle Verfahren.

Der Begriff "Stereoisomere" umfasst Konformationsisomere und insbesondere Konfigurationsisomere, wie Enantiomere und Diastereoisomere.

Generell mit umfasst sind erfindungsgemäß alle stereoisomeren Formen der hierin beschriebenen Verbindungen, wie Konstitutionsisomere und insbesondere Stereoisomere und Gemische davon, wie z.B. optische Isomere oder geometrische Isomere, wie E- und Z- Isomere., sowie Kombinationen davon. Sind mehrere Asymmetriezentren in einem Molekül vorhanden, so umfasst die Erfindung sämtliche Kombinationen von unterschiedlichen Konformationen dieser Asymmetriezentren, wie z.B. Enantiomerenpaare.

Der Begriff "stereospezifisch" bedeutet, dass eines von mehreren möglichen Stereoisomeren einer erfindungsgemäß hergestellten Verbindung mit wenigstens eine Asymmetriezentrum durch die Wirkung eines erfindungsgemäßen Enzyms in hohem "Enatiomerenüberschuss" oder hoher "Enantiomerenreinheit", wie z.B. wenigstens 90%ee, insbesondere wenigstens 95 %ee, oder wenigstens 98 %ee, oder wenigstens 99 %ee produziert wird. Der ee% Wert wird nach folgender Formel berechnet:

$$ee\% = [X_A - X_B]/[X_A + X_B]*100,$$

worin $X_A$ und $X_B$ für den Molenbruch der Enantiomere A bzw B stehen.

Unter einer "Zyklase-Aktivität", die mit einem "Referenzsubstrat unter Standardbedingungen" bestimmt wurde, ist z.B. eine Enzymaktivität, welche die Bildung eines cyclischen Produkts aus einem nicht-cyclischen Substrat beschreibt. Standardbedingungen sind z.B. Substratkonzentrationen von 10 mM bis 0,2 M, insbesondere 15 bis 100mM, wie z.B. etwa 20 bis 25 mM; bei pH 4 bis 8, und bei Temperaturen von z.B. 15 bis 30 oder 20 bis 25 °C. Die Bestimmung kann dabei mit rekombinanten Zyklase-exprimierenden Zellen, aufgeschlossenen Zyklase-exprimierenden Zellen, Fraktionen davon oder angereichertem oder gereinigtem Zyklase-Enzym erfolgen. Insbesondere ist Referenzsubstrat ein Homofarnesylsäure der Formel (Ia); Standardbedingungen sind insbesondere etwa 20 bis 25 mM Homofarnesylsäure der Formel (Ia), bei 20 bis 25 °C und pH 4 - 6, wie etwa 4,5; wie auch in den Bespielen näher beschrieben.

"Ausbeute" und/oder der "Umsatz" einer erfindungsgemäßen Reaktion wird über einen definierten Zeitraum von beispielsweise 4, 6, 8, 10, 12, 16, 20, 24, 36 oder 48 Stunden bestimmt, in dem die Umsetzung von Homofarnesylsäure zu Sclareolid mittels der erfindungsgemäßen Zyklasen erfolgt. Insbesondere wird die Umsetzung unter genau definierten Bedingungen durchgeführt, von zum Beispiel 25, 30, 40, 50 oder 60°C. Insbesondere erfolgt die Bestimmung der Ausbeute und/oder des Umsatzes indem die Reaktion zur Umsetzung von Homofarnesylsäure zu Sclareolid mittels der erfindungsgemäßen Zyklasen bei 30°C über 16 Stunden durchgeführt wird.

Zur Bestimmung der Ausbeute und/oder des Umsatzes wird insbesondere eine 10 mM Homofarnesylsäure-Lösung mit einer Zyklase-Lösung umgesetzt, wobei das Enzym als Membran-Proteinextrakt Zyklase-exprimierender Zellen (isoliert z.B. gemäß [Ochs D.et al.; J Bacteriol (1992), 174: 298]) in einer Konzentration an Proteingehalt von 0,08 Gewichtsprozent vorliegt.

Eine erfindungsgemäße Zyklase kann auch dadurch gekennzeichnet sein, dass sie bei der Umsetzung von Homofarnesylsäure zu Sclareolid unter den gleichen Bedingungen die 2-, 3-, 4-, 5-, 6-, 7-, 8-, 9-, 10-, 11-, 12-, 13-, 14-, 15-, 16-, 17-, 18-, 19-, 20-, 21-, 22-, 23-, 24-, 25-, 26-, 27-, 28-, 29-, 30-, 31-, 32-, 33-, 34-, 35-, 36-, 37-, 38-, 39-, 40-, 41-, 42-, 43-, 44-, 45-, 46-, 47-, 48-, 49-, 50-, 51-, 52-, 53-, 54-, 55-, 56-, 57-, 58-, 59-, 60-, 61-, 62-, 63-, 64-, 65-, 66-, 67-, 68-, 69-, 70-, 71-, 72-, 73-, 74-, 75-, 76-, 77-, 78-, 79-, 80-, 81-, 82-, 83-, 84-, 85-, 86-, 87-, 88-, 89-, 90-, 91-, 92-, 93-, 94-, 95-, 96-, 97-, 98-, 99-, 100-, 200-, 500-, 1000-fache oder höhere Ausbeute und/oder des Umsatz im Vergleich zu der Squalen-Hopen-Zyklase (SHC) aus *Alicyclobacillus acidocaldarius* (ehem. *Bacillus acidocaldarius*) zeigt Der Begriff "Bedingungen" bezieht sich hier auf Reaktionsbedingungen wie Susbstratkonzentration, Enzymkonzentration, Umsetzungszeitraum und/oder Temperatur.

Der Begriff "Carbonsäure" umfasst sowohl die freie Säure als auch die Salzform davon, wie z.B. deren Alkali- oder Erdalkalimetall-Salze. Dies gilt entsprechend für alle hierin genannten Carbonsäuren, wie z.B. die Homofarnesylsäure

Der Begriff "etwa" bezeichnet eine potentielle Änderung von ± 25% eines angegebenen Wertes, insbesondere ± 15%, ± 10%, vorzugsweise ± 5%, ± 2% oder ± 1%.

Der Begriff "im Wesentlichen" umschreibt einen Wertebereich von etwa 80 bis einschließlich 100%, wie z.B. 85-99,9% , insbesondere 90 bis 99,9%, vorzugsweise 95 bis 99,9% oder 98 bis 99,9% vor allem 99 bis 99,9%.

Sofern keine abweichenden Angaben gemacht werden gelten hierin folgende allgemeine chemische Definitionen:

"Alkyl" steht für gesättigte, geradkettige oder verzweigte, insbesondere geradkettige Kohlenwasserstoffreste mit 1 bis 6 insbesondere1 bis 4 Kohlenstoffatomen, z. B.

Methyl, Ethyl, Propyl, 1-Methylethyl, Butyl, 1-Methyl-propyl, 2-Methylpropyl, und 1,1-Dimethylethyl als beispielhafte Vertreter für $C_1$-$C_4$-Alkyl; sowie Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; insbesondere Methyl, Ethyl, n-Propyl und n-Butyl.

**B. Spezielle Ausgestaltungen der Erfindung**

Die vorliegende Erfindung betrifft insbesondere folgende spezielle Ausführungsformen:

1. Verfahren zur biokatalytischen Herstellung einer Verbindung der allgemeinen Formel II

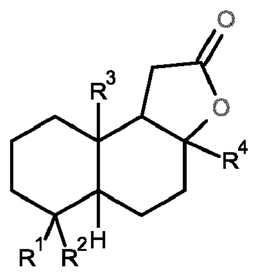

(II)

worin $R^1$, $R^2$, $R^3$ und $R^4$ unabhängig voneinander für H oder $C_1$-$C_4$-Alkyl, insbesondere Methyl oder Ethyl, vorzugsweise Methyl, stehen,
in stereoisomerenreiner Form oder als Gemisch von Stereoisomeren,
wobei man die Verbindung mit einem Protein, insbesondere einem Protein mit Zyklase-Aktivität, in Kontakt bringt, das zur Zyklisierung einer mehrfach ungesättigten Carbonsäure, insbesondere von Homofarnesylsäure, befähigt ist.

2. Verfahren nach Ausführungsform 1, wobei man die Verbindung der Formel I mit einem Protein in Kontakt bringt, das die enzymatische Aktivität einer Squalen-Hopen-Zyklase (SHC) aufweist.

3. Verfahren nach Ausführungsform 1 oder 2 wobei man als Substrat eine mehrfach ungesättigte Carbonsäure der allgemeinen Formel I,

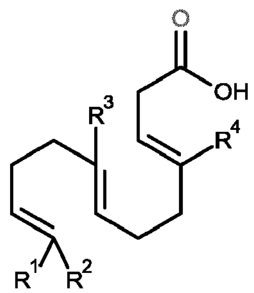

(I)

worin $R^1$, $R^2$, $R^3$ und $R^4$ die oben angegebenen Bedeutungen besitzen, insbesondere in im wesentlichen stereoisiomerenreiner Form, einsetzt.

4. Verfahren nach einer der vorherigen Ausführungsformen, wobei man Homofarnesylsäure der Formel Ia

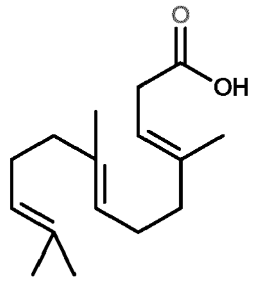

(Ia)

als Edukt einsetzt, insbesondere in im wesentlichen stereoisiomerenreiner Form, vorzugsweise in einem Anteil der (3E,7E)-Homofarnesylsaüre von wenigsten 70 mol%, besonders bevorzugt wenigsten 75, 80 oder 85 mol%, am meisten bevorzugt 90, 95 oder 99 oder 99,9 mol%, bezogen auf die Gesamtmenge an enthaltenen Homofarnesylsäure-Isomeren.

5. Verfahren nach Ausführungsform 4, wobei man Sclareolid der Formel IIa

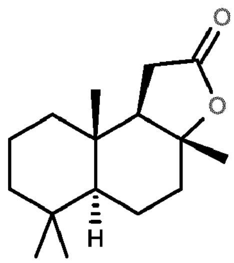

(IIa)

in stereoisomerenreiner Form oder als Gemisch von Stereoisomeren erhält.

6. Verfahren nach einer der vorherigen Ausführungsformen, wobei die SHC ausgewählt ist unter

a) Proteinen, umfassend ein Polypeptid mit einer Aminosäuresequenz gemäß SEQ ID NO: 2
b) durch Deletion, Insertion, Substitution, Addition, Inversion oder einer Kombination von Proteinen gemäß a) abgeleiteten Proteinen, umfassend ein Polypeptid mit einer Sequenzidentität von wenigsten 45%, 50%, 55%, 60%, 65 %, 70%, insbesondere wenigstens 75% oder 80%, vorzugsweise wenigsten 85 %, wie z.B. wenigstens 90, 91, 92, 93, 94, 95, 96, 97,98 oder 99%, zur Aminosäuresequenz gemäß SEQ ID NO: 2; und
c) zu a) oder b) funktional äquivalenten Proteinen, welche die Zyklisierung von Homofarnesylsäure zu Sclareolid katalysieren.

7. Verfahren nach Ausführungsform 6, wobei das funktional äquivalente Protein ein Polypeptid mit einer Aminosäuresequenz umfasst, die ausgewählt ist unter

a) SEQ ID NO: 3 bis 326 und
b) einer davon durch Deletion, Insertion, Substitution, Addition, Inversion oder einer Kombination abgeleiteten Aminosäuresequenz mit einem Identitätsgrad von wenigstens 60%, 65 %, 70%, insbesondere wenigstens 75% oder 80%, vorzugsweise wenigsten 85 %, wie z.B. wenigstens 90, 91, 92, 93, 94, 95, 96, 97,98 oder 99%,

8. Verfahren nach einer der vorherigen Ausführungsformen, wobei die enzymatische Zyklase-Aktivität, insbesondere dir Aktivität der SHC, in einer Form vorliegt, ausgewählt unter:

a) einer freien, gegebenenfalls teilweise oder vollständig gereinigten natürlichen oder rekombinant hergestellten Zyklase,
b) Zyklase gemäß a) in immobilisierter Form;
c) ganzen Zellen, enthaltend mindestens eine Zyklase;
d) Zelllysaten oder Zellhomogenisaten von Zellen gemäß c).

9. Verfahren nach einer der vorherigen Ausführungsformen, wobei die Umsetzung in einphasigen wässrigen Systemen oder in zweiphasigen wässrig-organischen oder festflüssigem Systemen erfolgt.

10. Verfahren nach einer der vorherigen Ausführungsformen, wobei die Umsetzung bei einer Temperatur im Bereich von 0 bis 60°C, insbesondere 10 bis 50°C, vorzugsweise 20 bis 40°C und/oder einem pH-Wert im Bereich von 4 bis 8, insbesondere 5 bis 7 erfolgt.

11. Verfahren nach einer der vorherigen Ausführungsformen, wobei die SHC aus einem Mikroorganismus ausgewählt unter *Methylococcus capsalatus, Rhodopseudomonas palustris, Bradyrhizobium japonicum, Frankia spec., Streptomyces coelicolor* und insbesondere *Zymomonas mobilis* isoliert wird.

12. Verfahren nach einer der vorherigen Ausführungsformen, wobei die SHC aus einem die SHC überexprimierenden Mikroorganismus isoliert wird, welcher ausgewählt ist unter Bakterien der Gattung *Escherichia, Corynebacterium, Ralstonia, Clostridium, Pseudomonas, Bacillus, Zymomonas, Rhodobacter, Streptomyces, Burkholderia, Lactobacillus* und *Lactococcus.*

13. Verfahren nach einer der vorherigen Ausführungsformen, wobei die SHC aus transgenen die SHC überexprimierenden Bakterien der Spezies *Escherichia coli, Pseudomonas putida, Burkholderia glumae, Streptomyces lividans, Streptomyces coelicolor* und *Zymomonas mobilis* isoliert wird.

14. Verfahren nach einer der vorherigen Ausführungsformen, wobei die Umsetzung in batch-, fed-batch oder kontinuierlicher Fahrweise erfolgt.

15. Verfahren nach einer der vorhergehenden Ausführungsformen, wobei man die biokatalytische Umsetzung unter wenigstens einer der folgenden Bedingungen durchführt:

a) bei einem pH Wert des Reaktionsmediums im Bereich von etwa 4 bis 5,9 oder 5,8 oder 4,5 bis 5,8, insbesondere 4,5 bis 5,5 oder 5 bis 5,5;
b) bei einer Substratkonzentration von mindesten 15 mM, wie z.B. bis zu 100 mM insbesondere bis zu 50 mM, insbesondere 15 bis 30 mM, vor allem 15 bis 25 mM;
c) bei einer Enzymkonzentration von wenigstens 5mg/ml; insbesondere 5 bis 100, vorzugsweise 10 bis 50 oder 15 bis 40 oder 15 bis 30 mg/ml
d) bei einer Reaktionstemperatur im Bereich von 32 bis 40 °C insbesondere 35 bis 38°C;
e) in einem Citrat-Puffer, insbesondere Natrium-Citrat-Puffer, insbesondere enthaltend 1 bis 20 mM oder 5 bis 10 mM $MgCl_2$
f) bei einer Pufferkonzentration von etwa 10 bis 100, insbesondere 20 bis 50 mM.
Bevorzugt werden die erfindungsgemöße Verfahren unter gleichzeitiger Verwirklichung der obigen Bedingungen a) bis b) oder a) bis c) oder a) bis d) oder a) bis e) oder a) bis f) durchgeführt. Dabei sind jegliche Kombinationen von Parameterbereichen unabhängig vom jeweiligen Bevorzugungsgrad einzelner Bereiche Teil der vorliegenden Offenbarung.

16. Verfahren zur Herstellung von 3a,6,6,9a-Tetramethyl-dodecahydronaphto[2,1-b]furan (Ambroxid), wobei man

a) Homofarnesylsäure nach einem Verfahren gemäß einem der Ansprüche 1 bis 13 zu Sclareolid umsetzt;
b) das Produkt aus Stufe a) in an sich bekannter Weise chemisch zu Ambroxdiol reduziert und
c) Ambroxidol aus Stufe b) in an sich bekannter Weise chemisch zu Ambroxid cyclisiert.

17. Verfahren nach Ausführungsform 16, wobei Ambroxid das (-)- Ambroxid ((3aR,5aS,9aS,9bR)-3a,6,6,9a-Tetramethyl-dodecahydronaphto[2,1-b]furan [CAS 6790-58-5]) ist.

## C. Weitere Ausgestaltungen der Erfindung

### 1. Besonders geeignete Zyklase-Sequenzen

Erfindungsgemäß brauchbare Zyklasen) sind SHCs deren SEQ ID NO für die entsprechende Wildtypsequenz, Quellorganismus und Genbank-Referenznummer, sind in folgender Tabelle zusammengefasst.

| S_ID DB | SEQ ID NO | Organism | GI-Nr. der Referenz Sequenzen |
|---|---|---|---|
| s1 | seq_ID 2 | ***Zymomonas mobilis*** | AAV90172.1 |
| s20 | seq_ID 3 | ***Streptomyces coelicolor*** | CAB39697.1 |
| s911 | seq_ID 4 | ***Acetobacter pasteurianus*** | BAH99456.1 |
| s2 | seq_ID 5 | ***Bradyrhizobium sp.*** | ABQ33590.1 |
| s940 | seq_ID 6 | ***Zymomonas mobilis*** | EER62728.1 |
| s949 | seq_ID 7 | ***Acidithiobacillus caldus*** | EET25937.1 |
| s167 | seq_ID 8 | ***Acidithiobacillus ferrooxidans*** | ACH84004.1 |
| s41 | seq_ID 9 | ***Acidobacterium capsulatum*** | ACO34244.1 |
| s36 | seq_ID 10 | ***Acidothermus cellulolyticus*** | ABK53469.1 |
| s83 | seq_ID 11 | ***Adiantum capillus-veneris*** | BAF93209.1 |
| s143 | seq_ID 12 | ***Ajellomyces capsulatus*** | EDN09769.1 |
| s995 | seq_ID 13 | ***Ajellomyces capsulatus*** | EER40510.1 |
| s163 | seq_ID 14 | ***Ajellomyces capsulatus*** | EEH02950.1 |
| s13 | seq_ID 15 | ***Alicyclobacillus acidocaldarius*** | EED08231.1 |
| s14 | seq_ID 16 | ***Alicyclobacillus acidocaldarius*** | P33247.4 |
| s1193 | seq_ID 17 | ***Alicyclobacillus acidocaldarius*** | AAT70690.1 |
| s21 | seq_ID 18 | ***Alicyclobacillus acidoterrestris*** | CAA61950.1 |
| s1189 | seq_ID 19 | ***Alicyclobacillus acidoterrestris*** | AAT70691.1 |
| s51 | seq_ID 20 | ***Anabaena variabilis*** | ABA24268.1 |
| s76 | seq_ID 21 | ***Anaeromyxobacter sp.*** | ABS28257.1 |
| s159 | seq_ID 22 | ***Aspergillus clavatus*** | EAW07713.1 |
| s131 | seq_ID 23 | ***Aspergillus flavus*** | EED48353.1 |
| s176 | seq_ID 24 | ***Aspergillus fumigatus*** | EDP50814.1 |
| s126 | seq_ID 25 | ***Aspergillus fumigatus*** | EAL84865.1 |
| s178 | seq_ID 26 | ***Aspergillus fumigatus*** | EAL86291.2 |
| s121 | seq_ID 27 | ***Aspergillus niger*** | CAK43501.1 |
| s115 | seq_ID 28 | ***Aspergillus niger*** | CAK45506.1 |
| s124 | seq_ID 29 | ***Aspergillus oryzae*** | BAE63941.1 |
| s119 | seq_ID 30 | ***Azotobacter vinelandii*** | EAM07611.1 |
| s223 | seq_ID 31 | ***Bacillus amyloliquefaciens*** | ABS74269.1 |
| s221 | seq_ID 32 | ***Bacillus anthracis*** | AAP27368.1 |
| s976 | seq_ID 33 | ***Bacillus cereus*** | EEK66523.1 |
| s225 | seq_ID 34 | ***Bacillus cereus*** | EAL12758.1 |
| s972 | seq_ID 35 | ***Bacillus cereus*** | EEL44583.1 |
| s977 | seq_ID 36 | ***Bacillus cereus*** | EEK43841.1 |
| s985 | seq_ID 37 | ***Bacillus cereus*** | EEK82938.1 |
| s988 | seq_ID 38 | ***Bacillus cereus*** | EEK99528.1 |
| s981 | seq_ID 39 | ***Bacillus cereus*** | EEK77935.1 |
| s987 | seq_ID 40 | ***Bacillus cereus*** | EEL81079.1 |
| s960 | seq_ID 41 | ***Bacillus cereus*** | EEK88307.1 |
| s979 | seq_ID 42 | ***Bacillus cereus*** | EEL63943.1 |
| s974 | seq_ID 43 | ***Bacillus cereus*** | EEL59884.1 |
| s956 | seq_ID 44 | ***Bacillus cereus*** | EEL69857.1 |
| s951 | seq_ID 45 | ***Bacillus cereus*** | EEL92663.1 |

(fortgesetzt)

| S_ID DB | SEQ ID NO | Organism | GI-Nr. der Referenz Sequenzen |
|---|---|---|---|
| s986 | seq_ID 46 | ***Bacillus cereus*** | EEL49968.1 |
| s227 | seq_ID 47 | ***Bacillus cereus*** | AAU 16998.1 |
| s224 | seq_ID 48 | ***Bacillus cereus*** | AAS42477.1 |
| s212 | seq_ID 49 | ***Bacillus cereus*** | ACK95843.1 |
| s289 | seq_ID 50 | ***Bacillus coahuilensis*** | 205373680 |
| s219 | seq_ID 51 | ***Bacillus cytotoxicus*** | ABS22481.1 |
| s230 | seq_ID 52 | ***Bacillus licheniformis*** | AAU23777.1 |
| s955 | seq_ID 53 | ***Bacillus mycoides*** | EEL98438.1 |
| s990 | seq_ID 54 | ***Bacillus mycoides*** | EEM04821.1 |
| s989 | seq_ID 55 | ***Bacillus pseudomycoides*** | EEM16144.1 |
| s247 | seq_ID 56 | ***Bacillus pumilus*** | ABV62529.1 |
| s250 | seq_ID 57 | ***Bacillus pumilus*** | EDW21137.1 |
| s249 | seq_ID 58 | ***Bacillus sp.*** | EAR64404.1 |
| s218 | seq_ID 59 | ***Bacillus sp.*** | EDL66148.1 |
| s241 | seq_ID 60 | ***Bacillus subtilis*** | Q796C3.1 |
| s284 | seq_ID 61 | ***Bacillus subtilis*** | AAB84441.1 |
| s215 | seq_ID 62 | ***Bacillus thuringiensis*** | ABK86448.1 |
| s984 | seq_ID 63 | ***Bacillus thuringiensis*** | EEM21409.1 |
| s957 | seq_ID 64 | ***Bacillus thuringiensis*** | EEM82653.1 |
| s980 | seq_ID 65 | ***Bacillus thuringiensis*** | EEM52372.1 |
| s961 | seq_ID 66 | ***Bacillus thuringiensis*** | EEM27851.1 |
| s969 | seq_ID 67 | ***Bacillus thuringiensis*** | EEM40716.1 |
| s959 | seq_ID 68 | ***Bacillus thuringiensis*** | EEM46814.1 |
| s965 | seq_ID 69 | ***Bacillus thuringiensis*** | EEM94969.1 |
| s202 | seq_ID 70 | ***Bacillus weihenstephanensis*** | ABY44436.1 |
| s63 | seq_ID 71 | ***Bacterium Ellin514*** | EEF57225.1 |
| s72 | seq_ID 72 | ***Bacterium Ellin514*** | EEF59508.1 |
| s87 | seq_ID 73 | ***Beijerinckia indica*** | ACB96717.1 |
| s69 | seq_ID 74 | ***Blastopirellula marina*** | EAQ81955.1 |
| s543 | seq_ID 75 | ***Blastopirellula marina*** | EAQ78122.1 |
| s156 | seq_ID 76 | ***Bradyrhizobium japonicum*** | CAA60250.1 |
| s938 | seq_ID 77 | ***Acetobacter pasteurianus*** | BAH98349.1 |
| s3 | seq_ID 78 | ***Bradyrhizobium sp.*** | CAL79893.1 |
| s201 | seq_ID 79 | ***Brevibacillus brevis*** | BAH44778.1 |
| s148 | seq_ID 80 | ***Burkholderia ambifaria*** | EDT05097.1 |
| s158 | seq_ID 81 | ***Burkholderia ambifaria*** | EDT37649.1 |
| s149 | seq_ID 82 | ***Burkholderia ambifaria*** | ACB68303.1 |
| s100 | seq_ID 83 | ***Burkholderia ambifaria*** | EDT42454.1 |
| s146 | seq_ID 84 | ***Burkholderia cenocepacia*** | EAY66961.1 |
| s139 | seq_ID 85 | ***Burkholderia cenocepacia*** | ACA95661.1 |
| s147 | seq_ID 86 | ***Burkholderia cenocepacia*** | CAR57099.1 |
| s95 | seq_ID 87 | ***Burkholderia cenocepacia*** | CAR56694.1 |
| s102 | seq_ID 88 | ***Burkholderia dolosa*** | EAY71311.1 |
| s941 | seq_ID 89 | ***Burkholderia glumae*** | ACR32572.1 |
| s945 | seq_ID 90 | ***Burkholderia glumae*** | ACR30752.1 |
| s132 | seq_ID 91 | ***Burkholderia graminis*** | EDT12320.1 |
| s104 | seq_ID 92 | ***Burkholderia mallei*** | ABM48844.1 |
| s140 | seq_ID 93 | ***Burkholderia multivorans*** | ABX19650.1 |
| s116 | seq_ID 94 | ***Burkholderia multivorans*** | ABX16859.1 |

(fortgesetzt)

| S_ID DB | SEQ ID NO | Organism | GI-Nr. der Referenz Sequenzen |
|---|---|---|---|
| s91 | seq_ID 95 | ***Burkholderia oklahomensis*** | 167567074 |
| s111 | seq_ID 96 | ***Burkholderia phymatum*** | ACC73258.1 |
| s127 | seq_ID 97 | ***Burkholderia phytofirmans*** | ACD21317.1 |
| s120 | seq_ID 98 | ***Burkholderia pseudomallei*** | EEC32728.1 |
| s137 | seq_ID 99 | ***Burkholderia sp.*** | EEA03553.1 |
| s144 | seq_ID 100 | ***Burkholderia sp.*** | ABB06563.1 |
| s98 | seq_ID 101 | ***Burkholderia sp.*** | ABB10136.1 |
| s944 | seq_ID 102 | ***Burkholderia sp. CCGE1002*** | EFA54357.1 |
| s89 | seq_ID 103 | ***Burkholderia thailandensis*** | 167840988 |
| s113 | seq_ID 104 | ***Burkholderia thailandensis*** | 167617352 |
| s154 | seq_ID 105 | ***Burkholderia ubonensis*** | 167589807 |
| s93 | seq_ID 106 | ***Burkholderia ubonensis*** | 167584986 |
| s96 | seq_ID 107 | ***Burkholderia vietnamiensis*** | ABO56791.1 |
| s150 | seq_ID 108 | ***Burkholderia xenovorans*** | ABE35912.1 |
| s54 | seq_ID 109 | ***Candidatus koribacter*** | ABF40741.1 |
| s171 | seq_ID 110 | ***Candidatus Kuenenia*** | CAJ71215.1 |
| s79 | seq_ID 111 | ***Candidatus Solibacter*** | ABJ82180.1 |
| s99 | seq_ID 112 | ***Candidatus Solibacter*** | ABJ82254.1 |
| s917 | seq_ID 113 | ***Catenulispora acidiphila*** | ACU75510.1 |
| s65 | seq_ID 114 | ***Chthoniobacter flavus*** | EDY15838.1 |
| s637 | seq_ID 115 | ***Chthoniobacter flavus*** | EDY22035.1 |
| s38 | seq_ID 116 | ***Crocosphaera watsonii*** | EAM53094.1 |
| s186 | seq_ID 117 | ***Cupriavidus taiwanensis*** | CAQ72562.1 |
| s32 | seq_ID 118 | ***Cyanothece sp.*** | ACB53858.1 |
| s40 | seq_ID 119 | ***Cyanothece sp.*** | ACK71719.1 |
| s30 | seq_ID 120 | ***Cyanothece sp.*** | EDY02410.1 |
| s29 | seq_ID 121 | ***Cyanothece sp.*** | ACK66841.1 |
| s47 | seq_ID 122 | ***Cyanothece sp.*** | EDX97382.1 |
| s35 | seq_ID 123 | ***Cyanothece sp.*** | EAZ91809.1 |
| s39 | seq_ID 124 | ***Cyanothece sp.*** | ACL45896.1 |
| s925 | seq_ID 125 | ***Cyanothece sp. PCC 8802*** | ACV02092.1 |
| s64 | seq_ID 126 | ***Desulfovibrio salexigens*** | EEC62384.1 |
| s74 | seq_ID 127 | ***Dryopteris crassirhizoma*** | BAG68223.1 |
| s59 | seq_ID 128 | ***Frankia alni*** | CAJ61140.1 |
| s48 | seq_ID 129 | ***Frankia alni*** | CAJ60090.1 |
| s56 | seq_ID 130 | ***Frankia sp.*** | ABD10207.1 |
| s60 | seq_ID 131 | ***Frankia sp.*** | ABW15063.1 |
| s31 | seq_ID 132 | ***Frankia sp.*** | ABW14125.1 |
| s948 | seq_ID 133 | ***Frankia sp. Eul1c*** | EFA59873.1 |
| s919 | seq_ID 134 | ***Frankia sp. Eul1c*** | EFA59089.1 |
| s628 | seq_ID 135 | ***Gemmata obscuriglobus*** | 168700710 |
| s209 | seq_ID 136 | ***Geobacillus sp.*** | EED61885.1 |
| s206 | seq_ID 137 | ***Geobacillus sp.*** | EDY05760.1 |
| s964 | seq_ID 138 | ***Geobacillus sp. Y412MC52*** | EEN95021.1 |
| s993 | seq_ID 139 | ***Geobacillus sp. Y412MC61*** | ACX79399.1 |
| s205 | seq_ID 140 | ***Geobacillus thermodenitrificans*** | ABO67242.1 |
| s15 | seq_ID 141 | ***Geobacter bemidjiensis*** | ACH40355.1 |
| s8 | seq_ID 142 | ***Geobacter lovleyi*** | ACD95949.1 |
| s62 | seq_ID 143 | ***Geobacter metallireducens*** | ABB30662.1 |

(fortgesetzt)

| S_ID DB | SEQ ID NO | Organism | GI-Nr. der Referenz Sequenzen |
|---|---|---|---|
| s12 | seq_ID 144 | ***Geobacter metallireducens*** | ABB33038.1 |
| s73 | seq_ID 145 | ***Geobacter sp.*** | ACM21577.1 |
| s10 | seq_ID 146 | ***Geobacter sp.*** | EDV72707.1 |
| s11 | seq_ID 147 | ***Geobacter p.*** | ACM22003.1 |
| s913 | seq_ID 148 | ***Geobacter sp. M18*** | EET34621.1 |
| s914 | seq_ID 149 | ***Geobacter sp. M21*** | ACT16952.1 |
| s58 | seq_ID 150 | ***Geobacter sulfurreducens*** | AAR36453.1 |
| s7 | seq_ID 151 | ***Geobacter sulfurreducens*** | AAR34018.1 |
| s9 | seq_ID 152 | ***Geobacter uraniireducens*** | ABQ25226.1 |
| s46 | seq_ID 153 | ***Gloeobacter violaceus*** | BAC91998.1 |
| s67 | seq_ID 154 | ***Gluconacetobacter diazotrophicus*** | ACI51585.1 |
| s165 | seq_ID 155 | ***Gluconacetobacter diazotrophicus*** | CAP55563.1 |
| s68 | seq_ID 156 | ***Gluconobacter oxydans*** | AAW61994.1 |
| s80 | seq_ID 157 | ***Granulibacter bethesdensis*** | ABI63005.1 |
| s937 | seq_ID 158 | ***Hyphomicrobium denitrificans*** | EET65847.1 |
| s932 | seq_ID 159 | ***Leptospirillum ferrodiazotrophum*** | EES53667.1 |
| s24 | seq_ID 160 | ***Leptospirillum rubarum*** | EAY57382.1 |
| s25 | seq_ID 161 | ***Leptospirillum sp.*** | EDZ38599.1 |
| s174 | seq_ID 162 | ***Magnaporthe grisea*** | EDK02551.1 |
| s153 | seq_ID 163 | ***Magnetospirillum magnetotacticum*** | 46203107 |
| s49 | seq_ID 164 | ***Methylacidiphilum infernorum*** | ACD82457.1 |
| s169 | seq_ID 165 | ***Methylobacterium chloromethanicum*** | ACK83067.1 |
| s75 | seq_ID 166 | ***Methylobacterium chloromethanicum*** | ACK86232.1 |
| s946 | seq_ID 167 | ***Methylobacterium extorquens*** | CAX24364.1 |
| s141 | seq_ID 168 | ***Methylobacterium nodulans*** | ACL61886.1 |
| s152 | seq_ID 169 | ***Methylobacterium populi*** | ACB79998.1 |
| s162 | seq_ID 170 | ***Methylobacterium radiotolerans*** | ACB27373.1 |
| s180 | seq_ID 171 | ***Methylobacterium sp.*** | ACA20611.1 |
| s175 | seq_ID 172 | ***Methylocella silvestris*** | ACK52150.1 |
| s181 | seq_ID 173 | ***Methylococcus capsulatus*** | CAA71098.1 |
| s55 | seq_ID 174 | ***Microcystis aeruginosa*** | CAO86472.1 |
| s101 | seq_ID 175 | ***Neosartorya fischeri*** | EAW20752.1 |
| s129 | seq_ID 176 | ***Nitrobacter hamburgensis*** | ABE63461.1 |
| s161 | seq_ID 177 | ***Nitrobacter sp.*** | EAQ34404.1 |
| s160 | seq_ID 178 | ***Nitrobacter winogradskyi*** | ABA05523.1 |
| s157 | seq_ID 179 | ***Nitrococcus mobilis*** | EAR22397.1 |
| s164 | seq_ID 180 | ***Nitrosococcus oceani*** | ABA57818.1 |
| s170 | seq_ID 181 | ***Nitrosomonas europaea*** | CAD85079.1 |
| s173 | seq_ID 182 | ***Nitrosomonas eutropha*** | ABI59752.1 |
| s943 | seq_ID 183 | ***Nitrosomonas sp. AL212*** | EET32702.1 |
| s142 | seq_ID 184 | ***Nitrosospira multiformis*** | ABB75845.1 |
| s52 | seq_ID 185 | ***Nostoc punctiforme*** | ACC84529.1 |
| s45 | seq_ID 186 | ***Nostoc sp.*** | BAB72732.1 |
| s122 | seq_ID 187 | ***Oligotropha carboxidovorans*** | ACI93782.1 |
| s233 | seq_ID 188 | ***Paenibacillus sp.*** | EDS49994.1 |
| s991 | seq_ID 189 | ***Paenibacillus sp. JDR-2*** | ACS99948.1 |
| s950 | seq_ID 190 | ***Paenibacillus sp. oral taxon 786*** | EES74793.1 |
| s1280 | seq_ID 191 | ***Paramecium tetraurelia*** | 145542269 |
| s71 | seq_ID 192 | ***Pelobacter carbinolicus*** | ABA87701.1 |

(fortgesetzt)

| S_ID DB | SEQ ID NO | Organism | GI-Nr. der Referenz Sequenzen |
|---|---|---|---|
| s5 | seq_ID 193 | ***Pelobacter carbinolicus*** | ABA87615.1 |
| s66 | seq_ID 194 | ***Pelobacter propionicus*** | ABK98395.1 |
| s16 | seq_ID 195 | ***Pelobacter propionicus*** | ABK98811.1 |
| s136 | seq_ID 196 | ***Penicillium chrysogenum*** | CAP99707.1 |
| s936 | seq_ID 197 | ***Planctomyces limnophilus*** | EEO67214.1 |
| s1158 | seq_ID 198 | ***Planctomyces limnophilus*** | EEO68341.1 |
| s526 | seq_ID 199 | ***Planctomyces maris*** | EDL58855.1 |
| s992 | seq_ID 200 | ***Polypodiodes niponica*** | BAI48071.1 |
| s942 | seq_ID 201 | ***Polypodiodes niponica*** | BAI48070.1 |
| s1202 | seq_ID 202 | ***Populus trichocarpa*** | EEF12098.1 |
| s168 | seq_ID 203 | ***Ralstonia eutropha*** | AAZ64302.1 |
| s190 | seq_ID 204 | ***Ralstonia eutropha*** | CAJ96989.1 |
| s81 | seq_ID 205 | ***Ralstonia metallidurans*** | ABF11015.1 |
| s110 | seq_ID 206 | ***Ralstonia metallidurans*** | ABF11268.1 |
| s123 | seq_ID 207 | ***Rhizobium sp.*** | P55348.1 |
| s657 | seq_ID 208 | ***Rhodopirellula baltica*** | CAD74517.1 |
| s4 | seq_ID 209 | ***Rhodopseudomonas palustris*** | ABJ08391.1 |
| s130 | seq_ID 210 | ***Rhodopseudomonas palustris*** | CAA71101.1 |
| s155 | seq_ID 211 | ***Rhodopseudomonas palustris*** | ABD06434.1 |
| s97 | seq_ID 212 | ***Rhodopseudomonas palustris*** | ABD87279.1 |
| s135 | seq_ID 213 | ***Rhodopseudomonas palustris*** | ACF02757.1 |
| s84 | seq_ID 214 | ***Rhodospirillum rubrum*** | ABC20867.1 |
| s1279 | seq_ID 215 | ***Rubrobacter xylanophilus*** | ABG05671.1 |
| s915 | seq_ID 216 | ***Saccharomonospora viridis*** | ACU97316.1 |
| s42 | seq_ID 217 | ***Saccharopolyspora erythraea*** | CAM03596.1 |
| s82 | seq_ID 218 | ***Schizosaccharomyces japonicus*** | EEB08219.1 |
| s923 | seq_ID 219 | ***Sphaerobacter thermophilus*** | ACZ39437.1 |
| s924 | seq_ID 220 | ***Streptomyces albus*** | 239983547 |
| s23 | seq_ID 221 | ***Streptomyces avermitilis*** | BAC69361.1 |
| s44 | seq_ID 222 | ***Acaryochloris marina*** | ABW29816.1 |
| s921 | seq_ID 223 | ***Streptomyces filamentosus*** | 239945642 |
| s934 | seq_ID 224 | ***Streptomyces flavogriseus*** | EEW70811.1 |
| s920 | seq_ID 225 | ***Streptomyces ghanaensis*** | 239927462 |
| s922 | seq_ID 226 | ***Streptomyces griseoflavus*** | 256812310 |
| s28 | seq_ID 227 | ***Streptomyces griseus*** | BAG17791.1 |
| s926 | seq_ID 228 | ***Streptomyces hygroscopicus*** | 256775136 |
| s916 | seq_ID 229 | ***Streptomyces lividans*** | 256783789 |
| s33 | seq_ID 230 | ***Streptomyces peucetius*** | ACA52082.1 |
| s27 | seq_ID 231 | ***Streptomyces pristinaespiralis*** | EDY61772.1 |
| s933 | seq_ID 232 | ***Streptomyces scabiei*** | CBG68454.1 |
| s37 | seq_ID 233 | ***Streptomyces sp.*** | EDX25760.1 |
| s34 | seq_ID 234 | ***Streptomyces sp.*** | EDY46371.1 |
| s931 | seq_ID 235 | ***Streptomyces sp. AA4*** | 256668250 |
| s918 | seq_ID 236 | ***Streptomyces sp. C*** | 256770952 |
| s929 | seq_ID 237 | ***Streptomyces sp. Mg1*** | 254385931 |
| s928 | seq_ID 238 | ***Streptomyces sp. SPB74*** | 254379682 |
| s930 | seq_ID 239 | ***Streptomyces sp. SPB78*** | 256680470 |
| s26 | seq_ID 240 | ***Streptomyces sviceus*** | EDY55942.1 |
| s927 | seq_ID 241 | ***Streptomyces viridochromogenes*** | 256805984 |

(fortgesetzt)

| S_ID DB | SEQ ID NO | Organism | GI-Nr. der Referenz Sequenzen |
|---|---|---|---|
| s61 | seq_ID 242 | ***Synechococcus sp.*** | EDX84551.1 |
| s935 | seq_ID 243 | ***Synechococcus sp. PCC 7335*** | 254422098 |
| s53 | seq_ID 244 | ***Synechocystis sp.*** | BAA17978.1 |
| s22 | seq_ID 245 | ***Syntrophobacter fumaroxidans*** | ABK18414.1 |
| s6 | seq_ID 246 | ***Syntrophobacter fumaroxidans*** | ABK17672.1 |
| s912 | seq_ID 247 | ***Teredinibacter turnerae*** | ACR13362.1 |
| s57 | seq_ID 248 | ***Thermosynechococcus elongatus*** | BAC09861.1 |
| s43 | seq_ID 249 | ***Trichodesmium erythraeum*** | ABG50159.1 |
| s1178 | seq_ID 250 | ***Uncultured organism*** | ACA58560.1 |
| s1176 | seq_ID 251 | ***Uncultured organism*** | ABL07557.1 |
| s1165 | seq_ID 252 | ***Uncultured organism*** | ACA58559.1 |
| s1166 | seq_ID 253 | ***Uncultured organism*** | ACA58558.1 |
| s1168 | seq_ID 254 | ***Uncultured organism*** | ABL07560.1 |
| s1169 | seq_ID 255 | ***Uncultured organism*** | ABL07565.1 |
| s1170 | seq_ID 256 | ***Uncultured organism*** | ABL07566.1 |
| s1167 | seq_ID 257 | ***Uncultured organism*** | ACA58545.1 |
| s1171 | seq_ID 258 | ***Uncultured organism*** | ACA58535.1 |
| s1180 | seq_ID 259 | ***Uncultured organism*** | ACA58549.1 |
| s1179 | seq_ID 260 | ***Uncultured organism*** | ACA58554.1 |
| s1181 | seq_ID 261 | ***Uncultured organism*** | ACA58555.1 |
| s1182 | seq_ID 262 | ***Uncultured organism*** | ACA58556.1 |
| s1235 | seq_ID 263 | ***Uncultured organism*** | ACA58530.1 |
| s1188 | seq_ID 264 | ***Uncultured organism*** | ACA58534.1 |
| s1237 | seq_ID 265 | ***Uncultured organism*** | ACA58552.1 |
| s1223 | seq_ID 266 | *Uncultured organism* | ABL07558.1 |
| s1200 | seq_ID 267 | ***Uncultured organism*** | ABL07542.1 |
| s1236 | seq_ID 268 | ***Uncultured organism*** | ACA58539.1 |
| s1238 | seq_ID 269 | ***Uncultured organism*** | ACA58537.1 |
| s1233 | seq_ID 270 | ***Uncultured organism*** | ACA58543.1 |
| s1173 | seq_ID 271 | ***Uncultured organism*** | ABL07553.1 |
| s1241 | seq_ID 272 | ***Uncultured organism*** | ABL07540.1 |
| s1242 | seq_ID 273 | ***Uncultured organism*** | ABL07544.1 |
| s1225 | seq_ID 274 | ***Uncultured organism*** | ACA58557.1 |
| s1183 | seq_ID 275 | ***Uncultured organism*** | ACA58520.1 |
| s1197 | seq_ID 276 | ***Uncultured organism*** | ACA58524.1 |
| s1185 | seq_ID 277 | ***Uncultured organism*** | ACA58522.1 |
| s1190 | seq_ID 278 | ***Uncultured organism*** | ACA58525.1 |
| s1187 | seq_ID 279 | ***Uncultured organism*** | ACA58523.1 |
| s1184 | seq_ID 280 | ***Uncultured organism*** | ACA58521.1 |
| s1204 | seq_ID 281 | ***Uncultured organism*** | ACA58547.1 |
| s1221 | seq_ID 282 | ***Uncultured organism*** | ACA58544.1 |
| s1198 | seq_ID 283 | ***Uncultured organism*** | ACA58546.1 |
| s1226 | seq_ID 284 | ***Uncultured organism*** | ACA58527.1 |
| s1227 | seq_ID 285 | ***Uncultured organism*** | ABL07537.1 |
| s1232 | seq_ID 286 | ***Uncultured organism*** | ACA58510.1 |
| s1230 | seq_ID 287 | ***Uncultured organism*** | ACA58538.1 |
| s1229 | seq_ID 288 | ***Uncultured organism*** | ACA58542.1 |
| s1231 | seq_ID 289 | ***Uncultured organism*** | ACA58540.1 |
| s1207 | seq_ID 290 | ***Uncultured organism*** | ABL07564.1 |

(fortgesetzt)

| S_ID DB | SEQ ID NO | Organism | GI-Nr. der Referenz Sequenzen |
|---|---|---|---|
| s1212 | seq_ID 291 | ***Uncultured organism*** | ABL07563.1 |
| s1208 | seq_ID 292 | ***Uncultured organism*** | ABL07562.1 |
| s1209 | seq_ID 293 | ***Uncultured organism*** | ABL07559.1 |
| s1214 | seq_ID 294 | ***Uncultured organism*** | ABL07556.1 |
| s1216 | seq_ID 295 | ***Uncultured organism*** | ACA58528.1 |
| s1219 | seq_ID 296 | ***Uncultured organism*** | ACA58536.1 |
| s1192 | seq_ID 297 | ***Uncultured organism*** | ABL07533.1 |
| s1195 | seq_ID 298 | ***Uncultured organism*** | ABL07536.1 |
| s1174 | seq_ID 299 | ***Uncultured organism*** | ABL07545.1 |
| s1186 | seq_ID 300 | ***Uncultured organism*** | ABL07548.1 |
| s1196 | seq_ID 301 | ***Uncultured organism*** | ACA58561.1 |
| s1172 | seq_ID 302 | ***Uncultured organism*** | ABL07555.1 |
| s1194 | seq_ID 303 | ***Uncultured organism*** | ABL07541.1 |
| s1211 | seq_ID 304 | ***Uncultured organism*** | ABL07554.1 |
| s1220 | seq_ID 305 | ***Uncultured organism*** | ABL07547.1 |
| s1203 | seq_ID 306 | ***Uncultured organism*** | ABL07550.1 |
| s1199 | seq_ID 307 | ***Uncultured organism*** | ABL07551.1 |
| s1228 | seq_ID 308 | ***Uncultured organism*** | ACA58509.1 |
| s1201 | seq_ID 309 | ***Uncultured organism*** | ACA58514.1 |
| s1205 | seq_ID 310 | ***Uncultured*** *organism* | ABL07543.1 |
| s1206 | seq_ID 311 | ***Uncultured organism*** | ABL07534.1 |
| s1177 | seq_ID 312 | ***Uncultured organism*** | ABL07546.1 |
| s1210 | seq_ID 313 | ***Uncultured organism*** | ABL07535.1 |
| s1175 | seq_ID 314 | ***Uncultured organism*** | ABL07552.1 |
| s1191 | seq_ID 315 | ***Uncultured organism*** | ABL07549.1 |
| s1222 | seq_ID 316 | ***Uncultured organism*** | ACA58553.1 |
| s1244 | seq_ID 317 | ***Uncultured organism*** | ABL07539.1 |
| s1213 | seq_ID 318 | ***Uncultured organism*** | ACA58532.1 |
| s1239 | seq_ID 319 | ***Uncultured organism*** | ACA58548.1 |
| s1215 | seq_ID 320 | ***Uncultured organism*** | ABL07561.1 |
| s1240 | seq_ID 321 | ***Uncultured organism*** | ACA58533.1 |
| s1234 | seq_ID 322 | ***Uncultured organism*** | ABL07538.1 |
| s1224 | seq_ID 323 | ***Uncultured organism*** | ACA58541.1 |
| s1217 | seq_ID 324 | ***Uncultured organism*** | ACA58529.1 |
| s596 | seq_ID 325 | ***Verrucomicrobium spinosum*** | 171910093 |
| s70 | seq_ID 326 | ***Acidiphilium cryptum*** | ABQ30890.1 |

SEQ ID NO:2 ist die Aminosäuresequenz der Zyklase die hierin auch als Zm-SHC-1 bezeichnet wird.

**2. Weitere erfindungsgemäße Proteine/Enzymmutanten**

Die vorliegende Erfindung ist nicht auf die hierin konkret offenbarten Proteine mit Zyklase-Aktivität beschränkt, sondern erstreckt sich vielmehr auch auf funktionale Äquivalente davon.

"Funktionale Äquivalente" oder Analoga der konkret offenbarten Enzyme, insbesondere von SEQ ID NO: 2 bis 6, sind im Rahmen der vorliegenden Erfindung davon verschiedene Polypeptide, welche weiterhin die gewünschte biologische Aktivität, wie insbesondere Zyclase-Aktivität, besitzen.

So versteht man beispielsweise unter "funktionalen Äquivalenten" Enzyme und Mutanten, die in einem verwendeten Test auf "Zyclasee-Aktivität" im Sinne der Erfindung (d.h. mit einem Referenzsubstrat unter Standardbedingungen) eine um mindestens 1%, insbesondere um mindestens etwa 5 bis 10 % wie z.B. mindestens 10% oder mindestens 20 %, wie z.B. mindestens 50 % oder 75% oder 90 % höhere oder niedrigere Aktivität eines Enzyms, umfassend eine hierin konkret definierte Aminosäuresequenz (insbesondere SEQ ID NO: 2 bis 6) aufweisen.

Die Aktivitätsangaben für funktionale Äquivalente beziehen sich hierin, wenn nichts anderes angegeben ist, auf Aktivitätsbestimmungen, durchgeführt mittels eines Referenzsubstrates unter Standardbedingungen, wie hierin definiert.

Die "Zyklase-Aktivität" im Sinne der Erfindung kann mit Hilfe verschiedener bekannter Tests nachgewiesen werden. Ohne darauf beschränkt zu sein, sei ein Test unter Verwendung eines Referenzsubstrates, wie z. B. Homofarnesylsäure, unter Standardbedingungen, wie oben beschrieben und im experimentellen Teil erläutert, zu nennen.

Funktionale Äquivalente sind außerdem z.B. zwischen pH 4 bis 11 stabil und besitzen vorteilhaft ein pH-Optimum in einem Bereich von pH 5 bis 10, wie insbesondere 6,5 bis 9,5 oder 7 bis 8 oder etwa bei 7,5, sowie ein Temperaturoptimum im Bereich von 15°C bis 80°C oder 20°C bis 70°C, wie z.B. etwa 30 bis 60°C oder etwa 35 bis 45°C, wie etwa bei 40°C.

Unter "funktionalen Äquivalenten" versteht man erfindungsgemäß insbesondere auch "Mutanten", welche abgeleitet sind von SEQ ID NO:2 bis 326, insbesondere von SEQ ID NO: 2 bis 6, und in wenigstens einer Sequenzposition der oben genannten Aminosäuresequenzen eine andere als die konkret genannte Aminosäure aufweisen aber trotzdem eine der oben genannten biologischen Aktivitäten besitzen.

"Funktionale Äquivalente" umfassen die durch eine oder mehrere, wie z.B. 1 bis 50, 2 bis 30, 2 bis 15, 4 bis 12 oder 5 bis 10 Mutationen, wie Aminosäure-Additionen, -Substitutionen, - Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Nichtlimitierende Beispiele für geeignete Aminosäuresubstitutionen sind in folgender Tabelle zusammengefasst:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gin ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen

oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75% ins besondere wenigsten 85 %, wie z.B. 90, 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### 3. Nukleinsäuren und Konstrukte

#### 3.1 Nukleinsäuren

Gegenstand der Erfindung sind auch Nukleinsäuresequenzen, die für ein wie oben beschriebenes Enzym mit Cyclase-Aktivität kodieren.

Die vorliegende Erfindung betrifft auch Nukleinsäuren mit einem bestimmten Identitätsgrad zu den hierin beschriebenen konkreten Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:
Multiple alignment parameters:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighting | 0 |

Pairwise alignment parameter:

| | |
|---|---|
| FAST algorithm | on |
| K-tuple size | 1 |
| Gap penalty | 3 |
| Window size | 5 |
| Number of best diagonals | 5 |

Alternativ dazu kann die Identität auch nach Chenna, Ramu, Sugawara, Hideaki, Koike, Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden

Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten.

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinander folgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Gegenstand der Erfindung sind auch Derivate der konkret offenbarten oder ableitbaren Nukleinsäuresequenzen.

So können weitere erfindungsgemäße, für Cyclase-Mutanten kodierende Nukleinsäuresequenzen z.B. von SEQ ID NO:1 oder von den kodierenden Sequenzen zu SEQ ID NO: 2 bis 326, insbesondere SEQ ID NO: 2 bis 6, durch eine F486 oder F486-analoge Mutation abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstitutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen, für Zyklasen kodierende Nukleinsäuresequenzen abgeleitet von Sequenz SEQ ID NO: 1 oder von einer der kodierenden Sequenzen zu SEQ ID NO: 2 bis 326, insbesondere SEQ ID NO: 2 bis 6, sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der erfindungsgemäßen Nukleinsäuresequenzen, beispielsweise pilzliche oder bakterielle Homologe, verkürzte Sequenzen, Einzelstrang-DNA oder RNA der kodierenden und nichtkodierenden DNA-Sequenz, zu verstehen.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertion, Inversion und/oder Deletion verändert sein, ohne dass aber die Funktionalität bzw. Wirksamkeit der Promotoren beeinträchtigt sind. Des Weiteren können die Promotoren durch Veränderung ihrer Sequenz in ihrer Wirksamkeit erhöht oder komplett durch wirksamere Promotoren auch artfremder Organismen ausgetauscht werden.

### 3.2 Generierung funktionaler Mutanten

Dem Fachmann sind darüber hinaus Verfahren zur Erzeugung funktionaler Mutanten erfindungsgemäßer Enzyme bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der von diesen codierten Protein sind dem Fachmann seit langem geläufig, wie beispielsweise

- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcárel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);

- die SeSaM-Methode (Sequence Saturation Method), bei der bevorzugte Austausche durch die Polymerase verhindert werden. Schenk et al., Biospektrum, Vol. 3, 2006, 277-279
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der so genannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen, wie z.B. 1, 2, 3, 4 oder 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die erfindungsgemäßen Ergebnisse liefern auch wichtige Informationen in Bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

Ebenfalls sind Informationen ableitbar bezüglich Aminosäure-Sequenzpositionen, in deren Bereich Mutationen durchgeführt werden können, die voraussichtlich wenig Einfuß auf die Enzymaktivität haben sollten, und als potentielle "silent mutations bezeichnet werden können.

### 3.3 Konstrukte

Gegenstand der Erfindung sind außerdem, insbesondere rekombinante, Expressionskonstrukte, enthaltend unter der genetischen Kontrolle regulativer Nukleinsäuresequenzen eine für ein erfindungsgemäßes Polypeptid kodierende Nukleinsäuresequenz; sowie, insbesondere rekombinante, Vektoren, umfassend wenigstens eines dieser Expressionskonstrukte.

Unter einer "Expressionseinheit" wird erfindungsgemäß eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere eine für eine Zyklase kodierende Sequenz, z.B. SEQ ID NO: 1 oder kodierend für eine Zyklase gemäß SEQ ID NO: 2 bis 326 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, $lacI^{q-}$, T7-, T5-, T3-, gal-, trc-, ara-, rhaP ($rhaP_{BAD}$)SP6-, lambda-$P_R$-oder im lambda-$P_L$-Promotor enthalten, die vorteilhafterweise in gram-negativen Bakterien Anwendung finden. Weitere vorteilhafte Regulationssequenzen sind beispielsweise in den grampositiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, $pIN-III^{113}$-B1, $\lambda$gt11 oder pBdCI, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, $pGHlac^+$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäße Nukleinsäurekonstrukt

oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

## 4. Mikroorganismen

Je nach Zusammenhang kann unter dem Begriff "Mikroorganismus" der Wildtyp-Mikroorganismus oder ein genetisch veränderter, rekombinanter Mikroorganismus oder beides verstanden werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit Phenylethanol Dehydrogenase-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

## 5. Rekombinante Herstellung von erfindungsgemäßen Enzymen

Gegenstand der Erfindung sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C. Die Kultur wird so lange fortgesetzt, bis sich ein Maximum des gewünschten Produktes gebildet hat. Dieses Ziel wird normalerweise innerhalb von 10 Stunden

bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen werden. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, T. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

Für die Expression erfindungsgemäßer Mutanten kann auf die Beschreibung der Expression des Wildtypenzyms EbN1 und der dafür brauchbaren Expressionssysteme in der WO2005/108590 und der WO2006/094945, zurückgegriffen werden, worauf hiermit ausdrücklich Bezug genommen wird.

### 6. Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" " in K. Drauz und H. Waldmann, Enzyme Catalysis in Organic Synthesis 2002, Vol.III, 991-1032, Wiley-VCH, Weinheim beschrieben. Weitere Informationen zu Biotransformationen und Bioreaktoren zur Durchführung erfindungsgemäßer Verfahren findet man z.B. auch in Rehm et al (Ed) Biotechology, 2nd Edn, Vol 3, Chapter 17, VCH, Weinheim.

### 7. Enzymatische Cyclisierung von mehrfach ungesättigten Carbonsäuren

#### 7.1 Allgemein

Insbesondere wird das erfindungsgemäße Cyclisierungsverfahren in Gegenwart eines Enzyms durchgeführt, wobei das Enzym von einer Nukleinsäuresequenz gemäß SEQ ID NO:1 oder einem funktionalen Äquivalent davon kodiert wird, wobei die Nukleinsäuresequenz Bestandteil eines Genkonstrukts oder Vektors ist. Solche Genkonstrukte

oder Vektoren werden ausführlich in der internationalen Anmeldung PCT/EP2010/057696 auf den Seiten 16 bis 20 beschrieben, worauf hier ausdrücklich Bezug genommen wird.

Die Wirtszelle, die ein Genkonstrukt oder einen Vektor enthält, worin die Nukleinsäuresequenz enthalten ist, die das Enzym mit der gewünschten Aktivität kodiert, bezeichnet man auch als transgenen Organismus. Die Herstellung solche transgenen Organismen ist prinzipiell bekannt und wird beispielsweise in internationalen Anmeldung PCT/EP2010/057696 auf Seite 20 diskutiert, worauf hier ausdrücklich Bezug genommen wird.

Als transgene Organismen werden bevorzugt Zellen aus der Gruppe bestehend aus Bakterien, Cyanobakterien, Pilzen und Hefen ausgewählt. Bevorzugt ist die Zelle ausgewählt aus Pilzen der Gattung Pichia oder Bakterien der Gattungen Escherichia, Corynebacterium, Ralstonia, Clostridium, Pseudomonas, Bacillus, Zymomonas, Rhodobacter, Streptomyces, Burkholderia, Lactobacillus oder Lactococcus. Besonders bevorzugt ist die Zelle ausgewählt aus Bakterien der Spezies Escherichia coli, Pseudomonas putida, Burkholderia glumae, Streptomyces lividans, Streptomyces coelicolor oder Zymomonas mobilis.

Bevorzugt ist ein erfindungsgemäßes Verfahren, dadurch gekennzeichnet, dass das Enzym mit der Aktivität einer Homofarnesylsaäure-Cyclase von einem Gen kodiert wird, das aus einem Mikroorganismus isoliert wurde, ausgewählt unter Zymomonas mobilis, Methylococcus capsulatus, Rhodopseudomonas palustris, Bradyrhizobium japonicum, Frankia spec, Streptomyces coelicolor sowie Acetobacter pasteurianus. Besonders zu nennen sind die betreffenden Gene aus Zymomonas mobilis, Streptomyces coelicolor, Bradyrhizobium japonicum und Acetobacter pasteurianus isoliert.

Weiterhin bevorzugt ist ein erfindungsgemäßes Verfahren, dadurch gekennzeichnet, dass das Enzym mit der Cyclase-Aktivität von einem Mikroorganismus generiert wurde, der das Enzym überproduziert und der ausgewählt wurde aus der Gruppe der Mikroorganismen bestehend aus den Gattungen Escherichia, Corynebacterium, Ralstonia, Clostridium, Pseudomonas, Bacillus, Zymomonas, Rhodobacter, Streptomyces, Burkholderia, Lactobacillus und Lactococcus.

Besonders zu erwähnen ist ein erfindungsgemäßes Verfahren, dadurch gekennzeichnet dass das Enzym mit der Cyclase-Aktivität von transgenen Mikroorganismen der Spezies Escherichia coli, Pseudomonas putida, Burkholderia glumae, Corynebacterium glutamicum, Saccharomyces cerevisiae, Pichia pastoris, Streptomyces lividans, Streptomyces coelicolor, Bacillus subtilis oder Zymomonas mobilis erzeugt wurde, welche das Enzym mit der Cyclase-Aktivität überproduzieren.

Weitere Ausgestaltungen zur Durchführung des erfindungsgemäßen biokatalytischen Cyclisierungsverfahrens.

Das erfindungsgemäße Verfahren ist dadurch gekennzeichnet, dass das Enzym in wenigstens einer der folgenden Formen vorliegt:

a) freies, gegebenenfalls gereinigtes oder teilweise gereinigtes Polypeptid;
b) immobilisiertes Polypeptid;
c) aus Zellen isoliertes Polypeptid gemäß a) oder b);
d) ganze Zelle, gegebenenfalls ruhende oder wachsende Zellen, enthaltend mindestens ein solches Polypeptid;
e) Lysat oder Homogenisat der Zellen gemäß d).

Eine weitere Ausführungsform des erfindungsgemäßen Verfahrens ist dadurch gekennzeichnet, dass die Zellen Mikroorganismen sind, bevorzugt transgene Mikroorganismen exprimierend mindestens ein heterologes Nukleinsäuremolekül kodierend für ein Polypeptid mit der Cyclase-Aktivität.

Eine bevorzugte Ausführungsform des erfindungsgemäßen Verfahrens umfasst wenigsten die folgenden Schritte a), b) und d):

a) einen ein Enzym mit Zyklase-Aktivität produzierenden Mikroorganismus aus einer natürlichen Quelle zu isolieren oder rekombinant herzustellen,
b) diesen Mikroorganismus zu vermehren,
c) aus dem Mikroorganismus das Enzym mit Zyklase-Aktivität gegebenenfalls zu isolieren oder eine dieses Enzym enthaltende Proteinfraktion herzustellen, und
d) den Mikroorganismus gemäß Stufe b) oder das Enzym gemäß Stufe c) in ein Medium zu überführen, das Substrat, z.B. Homofarnesylsäure der allgemeinen Formel (Ia), enthält.

In dem erfindungsgemäßen Verfahren wird Substrat mit dem Enzym, das die Aktivität einer Zyklase besitzt, in einem Medium in Kontakt gebracht und/oder so inkubiert, dass eine Umsetzung des Substrats, wie z.B. von Homofarnesylsäure, zu Sclareolid in Gegenwart des Enzyms erfolgt. Bevorzugt handelt es sich bei dem Medium um ein wässriges Reaktionsmedium.

Der pH-Wert des wässrigen Reaktionsmediums, in dem das erfindungsgemäße Verfahren bevorzugt durchgeführt wird, wird dabei vorteilhaft zwischen pH 4 und 12, bevorzugt zwischen pH 4,5 und 9, besonders bevorzugt zwischen pH 5 und 8 gehalten.

Bei den wässrigen Reaktionsmedien handelt es sich vorzugsweise um gepufferte Lösungen, die in der Regel einen pH-Wert von vorzugsweise von 5 bis 8, aufweisen. Als Puffer kann ein Citrat-, Phosphat-, TRIS- (Tris(hydroxymethyl)-aminomethan) oder MES-Puffer (2-(N-Morpholino)ethansulfonsäure) verwendet werden. Ferner kann das Reaktionsmedium noch weitere Zusätze enthalten, wie z.B. Detergentien (beispielsweise Taurodeoxycholat).

Das Substrat, wie z.B. Homofarnesylsäure, wird vorzugsweise in einer Konzentration von 2 - 200mM, besonders bevorzugt von 5 - 25mM in die enzymatische Umsetzung eingesetzt und kann kontinuierlich oder diskontinuierlich nachgeführt werden.

Die enzymatische Cyclisierung erfolgt in der Regel bei einer Reaktionstemperatur unterhalb der Desaktivierungstemperatur des eingesetzten Enzyms und oberhalb von -10°C. Bevorzugt wird das erfindungsgemäße Verfahren bei einer Temperatur zwischen 0°C und 95°C, besonders bevorzugt bei einer Temperatur zwischen 15°C und 60°C, insbesondere zwischen 20 und 40°C, z.B. bei etwa 25 bis 30 °C durchgeführt.

Besonders bevorzugt ist ein erfindungsgemäßes Verfahren, wobei die Reaktion von Homofarnesylsäure zu Sclareolid bei einer Temperatur im Bereich von 20 bis 40 °C und/oder einem pH-Wert im Bereich von 4 bis 8 erfolgt.

Neben diesen einphasigen wässrigen Systemen werden in einer weiteren Variante der Erfindung auch zweiphasige Systeme eingesetzt. Dabei werden neben einer wässrigen Phase als zweite Phase, organische, nicht-wassermischbare Reaktionsmedien verwendet. Dadurch akkumulieren die Reaktionsprodukte in der organischen Phase. Nach der Reaktion ist das Produkt in der organische Phase leicht von der wässrigen Phase, die den Biokatalysator enthält, abtrennbar.

Bevorzug ist ein erfindungsgemäßes Verfahren, dadurch gekennzeichnet, dass die Umsetzung von Homofarnesylsäure in einphasigen wässrigen Systemen oder in zweiphasigen Systemen, oder die Umsetzung von schwerlöslichen Homofarnesylsäuresalzen in zweiphasigen Wässrig/festen Systemen erfolgt.

Das Reaktionsprodukt kann mit organischen Lösungsmitteln extrahiert werden und gegebenenfalls zur Aufreinigung destilliert werden.

Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Heptan, Methyl-tert-butylether, Diisopropylether, Tetrahydrofuran, Ethylacetat verwendet.

Die erfindungsgemäß eingesetzten Zyklasen können im erfindungsgemäßen Verfahren als freies oder immobilisiertes Enzym, wie oben bereits beschrieben, verwendet werden.

Für das erfindungsgemäße Verfahren können ruhende oder wachsende, freie oder immobilisierte Zellen verwendet werden, die für die Zyklase kodierenden Nukleinsäuren, Nukleinsäurekonstrukte oder Vektoren enthalten. Auch aufgeschlossene Zellen, wie Zelllysate oder Zellhomogenate, können verwendet werden. Unter aufgeschlossenen Zellen sind beispielsweise Zellen zu verstehen, die über eine Behandlung beispielsweise mit Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder teilweise gereinigte Enzyme können für das Verfahren verwendet werden.

Werden für das erfindungsgemäße Verfahren freie Organismen oder Enzyme verwendet, so werden diese vor der Extraktion zweckmäßigerweise abgetrennt, beispielsweise über eine Filtration oder Zentrifugation.

Das erfindungsgemäße Verfahren kann batchweise, semi-batchweise oder kontinuierlich betrieben werden.

### 7.2 Bevorzugte Umsetzung von Homofarnesylsäure zu Sclareolid

Die Erfindung betrifft insbesondere ein Verfahren zur Herstellung von Sclareolid, bei dem

a) Homo¬farnesy¬säure mit der Homofarnesol-Ambroxan-Zyklase in Kontakt gebracht und/oder inkubiert wird, und

b) Sclareolid isoliert wird.

In einer Ausführung der Erfindung wird Homo¬farnesy¬säure mit der Zyklase in einem Medium in Kontakt gebracht und/oder so inkubiert, dass eine Umsetzung von Homo¬farnesyl¬säure zu Sclareolid in Gegenwart der Zyklase erfolgt. Bevorzugt handelt es sich bei dem Medium um ein wässriges Reaktionsmedium. Bei den wässrigen Reaktionsmedien handelt es sich vorzugsweise um gepufferte Lösungen, die in der Regel einen pH-Wert von vorzugsweise von

5 bis 8, aufweisen. Als Puffer kann ein Citrat-, Phosphat-, TRIS (Tris(hydroxymethyl)-aminomethan), MES (2-(N-Morpholino)ethansulfonsäure)- Puffer verwendet werden. Ferner kann das Reaktionsmedium noch weitere Zusätze enthalten, wie z.B. Detergentien (beispielsweise Taurodeoxycholat).

Das Substrat wird vorzugsweise in einer Konzentration von 5 - 100mM, besonders bevorzugt von 15 - 25mM in die enzymatische Umsetzung eingesetzt und kann kontinuierlich oder diskontinuierlich nachgeführt werden.

Die enzymatische Zyklisierung erfolgt in der Regel bei einer Reaktionstemperatur unterhalb der Desaktivierungstemperatur der eingesetzten Zyklase und oberhalb von -10 °C. Besonders bevorzugt liegt sie im Bereich von 0 bis 100 °C, insbesondere von 15 bis 60 °C und speziell von 20 bis 40 °C, z.B. bei etwa 30 °C.

Das Reaktionsprodukt Sclareolid kann mit organischen Lösungsmitteln, ausgewählt aus der Gruppe der unten genannten, extrahiert werden und gegebenenfalls zur Aufreinigung destilliert werden.

Neben diesen einphasigen wässrigen Systemen werden in einer weiteren Variante der Erfindung auch zweiphasige Systeme eingesetzt. Dabei werden ionische Flüssigkeiten als zweite Phase, bevorzugt aber organische, nicht-wasser-mischbare Reaktionsmedien als zweite Phase verwendet. Dadurch akkumulieren die Reaktionsprodukte in der organischen Phase. Nach der Reaktion ist Ambroxan in der organische Phase leicht von der wässrigen Phase, die den Biokatalysator enthält, abtrennbar.

Unter nicht-wässrigen Reaktionsmedien werden Reaktionsmedien verstanden, die weniger als 1 Gew.-%, vorzugsweise weniger als 0,5 Gew.-% Wasser, bezogen auf das Gesamtgewicht des flüssigen Reaktionsmediums, enthalten. Insbesondere kann die Umsetzung in einem organischen Lösungsmittel durchgeführt werden.

Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Heptan, Methyl-*tert*-butylether, Diisopropylether, Tetrahydrofuran, Ethylacetat verwendet.

Die Umsetzung von Homo¬farnesy¬säure zu Sclareolid kann nicht nur in einphasigen wässrigen Systemen sondern auch in zweiphasigen Systemen stattfinden. Bei den zweiphasigen Systemen werden die oben genannten eingesetzt. Bevorzugt werden die oben genannten organische, nicht-wasser-mischbare Lösungsmittel als zweite Phase verwendet. Dadurch akkumulieren die Reaktionsprodukte in der organischen Phase. Nach der Reaktion ist Ambroxan in der organische Phase leicht von der wässrigen Phase, die den Biokatalysator enthält, abtrennbar.

Ein weiterer Gegenstand der vorliegenden Erfindung ist ein Verfahren zur biokatalytischen Herstellung von Sclareolid, dadurch gekennzeichnet, dass das Enzym ein Polypeptid ist, welches kodiert wird von einem Nukleinsäuremolekül umfassend mindestens ein Nukleinsäuremolekül ausgewählt aus der Gruppe bestehend aus:

a) Nukleinsäuremolekül, das ein Polypeptid kodiert, umfassend die in SEQ ID NO 2;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 45% zu den Sequenzen SEQ ID NO 2 aufweist;
d) Nukleinsäuremolekül nach (a) bis (c), das für ein funktional äquivalentes Polypeptid oder ein Fragment der Sequenz gemäß SEQ ID NO 2 kodiert;
e) Nukleinsäuremolekül, kodierend für ein funktional äquivalentes Polypeptid mit der Aktivität einer Homofarnesylsäure-Zyklase, das man erhält in dem man ein Nukleinsäuremolekül aus einer cDNA Bank oder aus genomischer DNA mittels der Primer gemäß SEQ ID NO: Nr. 327 und 328 amplifiziert, oder das Nukleinsäuremolekül durch *de novo* Synthese chemisch synthetisiert;
f) Nukleinsäuremolekül, kodierend für ein funktional äquivalentes Polypeptid mit der Aktivität einer Homofarnesylsäure-Zyklase, das unter stringenten Bedingungen mit einem Nukleinsäuremolekül gemäß (a) bis (c) hybridisiert;
g) Nukleinsäuremolekül kodierend für ein funktional äquivalentes Polypeptid mit der Aktivität einer Homofarnesylsäure-Zyklase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
h) Nukleinsäuremolekül, kodierend für ein funktional äquivalentes Polypeptid mit der Aktivität einer Homofarnesylsäure-Zyklase, wobei die Sequenz des Polypeptids eine Identität von mindestens 30% zu den Sequenzen SEQ ID NO 2 aufweist;
i) Nukleinsäuremolekül, kodierend für ein funktional äquivalentes Polypeptid mitder Aktivität einer Homofarnesylsäure-Zyklase, wobei das Polypeptid durch ein Nukleinsäuremolekül ausgewählt aus der Gruppe der in a) bis h)
j) Nukleinsäuremolekül, kodierend für ein funktional äquivalentes Polypeptid mitder Aktivität einer Homofarnesylsäure-Zyklase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.

Eine analoge oder ähnliche Bindungsstelle im Sinne der Erfindung ist eine konservierte Domäne oder Motiv der Aminosäurensequenz mit einer Homologie von 80%, besonders bevorzugt 85%, 86%, 87%, 88%, 89%, 90%, insbesondere 91%, 92%, 93%, 94%, 95%, 96%, 97%, 98% oder 99% oder 100%, welche die Bindung desselben Substrats, insbesondere Homo¬farnesy¬säure gewährleistet.

Bevorzugt weist das Nukleinsäuremolekül c) eine Identität mit der SEQ ID NO:1 von 15mindestens 50%, 60%, 65%, 70%, 75%, 80%, besonders bevorzugt 85%, 86%, 87%, 88%, 89%, 90%, insbesondere 91%, 92%, 93%, 94%, 95%, 96%, 97%, 98% oder 99% auf.

Ebenso weisen ein funktional äquivalentes Polypeptid eine Identität mit der SEQ ID NO: 2 von mindestens 50%, 60%, 65%, 70%, 75%, 80%, besonders bevorzugt 85%, 86%, 87%, 88%, 89%, 90%, insbesondere 91%, 92%, 93%, 94%, 95%, 96%, 97%, 98% oder 99% auf. Anstelle des Begriffs "Identität" kann auch der Begriff "homolog" oder "Homologie" gleichbedeutend verwendet werden.

Die Erfindung wird nun unter Bezugnahme auf folgende nichtlimitierende Beispiele näher beschrieben:

## Experimenteller Teil

Sofern keine speziellen Angaben in den folgenden Beispielen gemacht werden, gelten die folgenden allgemeinen Angaben.

### A. Allgemeine Angaben

Sämtliche eingesetzte Materialien und Mikroorganismen sind im Handel erhältliche Produkte.

Soweit nicht anderes angegeben wird, erfolgt die Klonierung und Expression von rekombinanten Proteinen nach Standardmethoden, wie z.B. in Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989 beschrieben.

### B. Beispiele

#### Beispiel 1 Klonierung der Zm-SHC und Expression in E. coli

Mit den Oligonukleotiden Zm-SHC_fw und Zm-SHC_rev kann das Gen der Zyklase aus *Zymomonas mobilis* amplifiziert werden.

| Primer: | | |
|---|---|---|
| Primer Nr. | Sequenz (5'->3') | Position |
| Zm-SHC_fw | gcgctgtttcatatgggtattgaca (SEQ ID NO:327) | N-Term-Primer |
| Zm-SHC_rev | gcgcttaccctggatcctcgaaaat (SEQ ID NO:328) | C-Term-Primer |

Je 100 ng der Primer Zm-SHC_fw und Zm-SHC_rev wurden equimolar gemischt. Die PCR mit genomischer DNA aus *Z. mobilis* (ATCC31821) wurde nach Herstellerangaben mit Pwo- Polymerase (Roche Applied Science) und dem folgenden Temperatur-Gradienten-Programmdurchgeführt: 95°C für 3 min; 30 Zyklen mit 95°C für 30 sec, 50°C für 30 sec, und 72°C für 3 min; 72°C für 10 min.; 4°C bis zur Verwendung. Das PCR-Produkt (~2.2 kB) wurde durch Agarosegel-Elektrophorese (1,2%E-Gel, Invitrogen) und Säulen-Chromatographie (GFX-Kit, Amersham Pharmacia) isoliert und anschließend sequenziert (Sequenzierprimer: Zm-SHC_fw und Zm-SHC_rev). Die erhaltene Sequenz entspricht der publizierten Sequenz.

Das PCR-Produkt wurde mit den Restriktionsendonukleasen *Nde*I und *Bam*HI verdaut und in entsprechend verdauten *p*DHE19.2-Vektor[9] ligiert. Die Sequenzierung der resultierenden Plasmide ergab die in SEQ ID NO:1 dargestellte Nukleinsäuresequenz. Die Korrespondierende Aminosäuresequenz ist im Folgenden dargestellt /(SEQ ID NO:2):

```
Met Gly Ile Asp Arg Met Asn Ser Leu Ser Arg Leu Leu Met Lys Lys
1               5                   10                  15

Ile Phe Gly Ala Glu Lys Thr Ser Tyr Lys Pro Ala Ser Asp Thr Ile
            20                  25                  30

Ile Gly Thr Asp Thr Leu Lys Arg Pro Asn Arg Arg Pro Glu Pro Thr
        35                  40                  45

Ala Lys Val Asp Lys Thr Ile Phe Lys Thr Met Gly Asn Ser Leu Asn
    50                  55                  60

Asn Thr Leu Val Ser Ala Cys Asp Trp Leu Ile Gly Gln Gln Lys Pro
65                  70                  75                  80

Asp Gly His Trp Val Gly Ala Val Glu Ser Asn Ala Ser Met Glu Ala
                85                  90                  95

Glu Trp Cys Leu Ala Leu Trp Phe Leu Gly Leu Glu Asp His Pro Leu
            100                 105                 110

Arg Pro Arg Leu Gly Asn Ala Leu Leu Glu Met Gln Arg Glu Asp Gly
        115                 120                 125

Ser Trp Gly Val Tyr Phe Gly Ala Gly Asn Gly Asp Ile Asn Ala Thr
    130                 135                 140

Val Glu Ala Tyr Ala Ala Leu Arg Ser Leu Gly Tyr Ser Ala Asp Asn
145                 150                 155                 160

Pro Val Leu Lys Lys Ala Ala Ala Trp Ile Ala Glu Lys Gly Gly Leu
                165                 170                 175
```

```
Lys Asn Ile Arg Val Phe Thr Arg Tyr Trp Leu Ala Leu Ile Gly Glu
            180                 185                 190

Trp Pro Trp Glu Lys Thr Pro Asn Leu Pro Pro Glu Ile Ile Trp Phe
        195                 200                 205

Pro Asp Asn Phe Val Phe Ser Ile Tyr Asn Phe Ala Gln Trp Ala Arg
    210                 215                 220

Ala Thr Met Val Pro Ile Ala Ile Leu Ser Ala Arg Arg Pro Ser Arg
225                 230                 235                 240

Pro Leu Arg Pro Gln Asp Arg Leu Asp Glu Leu Phe Pro Glu Gly Arg
                245                 250                 255

Ala Arg Phe Asp Tyr Glu Leu Pro Lys Lys Glu Gly Ile Asp Leu Trp
            260                 265                 270

Ser Gln Phe Phe Arg Thr Thr Asp Arg Gly Leu His Trp Val Gln Ser
        275                 280                 285

Asn Leu Leu Lys Arg Asn Ser Leu Arg Glu Ala Ala Ile Arg His Val
    290                 295                 300

Leu Glu Trp Ile Ile Arg His Gln Asp Ala Asp Gly Gly Trp Gly Gly
305                 310                 315                 320

Ile Gln Pro Pro Trp Val Tyr Gly Leu Met Ala Leu His Gly Glu Gly
                325                 330                 335

Tyr Gln Leu Tyr His Pro Val Met Ala Lys Ala Leu Ser Ala Leu Asp
            340                 345                 350

Asp Pro Gly Trp Arg His Asp Arg Gly Glu Ser Ser Trp Ile Gln Ala
        355                 360                 365

Thr Asn Ser Pro Val Trp Asp Thr Met Leu Ala Leu Met Ala Leu Lys
    370                 375                 380

Asp Ala Lys Ala Glu Asp Arg Phe Thr Pro Glu Met Asp Lys Ala Ala
385                 390                 395                 400

Asp Trp Leu Leu Ala Arg Gln Val Lys Val Lys Gly Asp Trp Ser Ile
                405                 410                 415

Lys Leu Pro Asp Val Glu Pro Gly Gly Trp Ala Phe Glu Tyr Ala Asn
            420                 425                 430

Asp Arg Tyr Pro Asp Thr Asp Asp Thr Ala Val Ala Leu Ile Ala Leu
        435                 440                 445

Ser Ser Tyr Arg Asp Lys Glu Glu Trp Gln Lys Lys Gly Val Glu Asp
    450                 455                 460

Ala Ile Thr Arg Gly Val Asn Trp Leu Ile Ala Met Gln Ser Glu Cys
465                 470                 475                 480

Gly Gly Trp Gly Ala Phe Asp Lys Asp Asn Asn Arg Ser Ile Leu Ser
                485                 490                 495

Lys Ile Pro Phe Cys Asp Phe Gly Glu Ser Ile Asp Pro Pro Ser Val
            500                 505                 510

Asp Val Thr Ala His Val Leu Glu Ala Phe Gly Thr Leu Gly Leu Ser
        515                 520                 525

Arg Asp Met Pro Val Ile Gln Lys Ala Ile Asp Tyr Val Arg Ser Glu
```

```
            530                 535                 540

        Gln Glu Ala Glu Gly Ala Trp Phe Gly Arg Trp Gly Val Asn Tyr Ile
        545                 550                 555                 560

        Tyr Gly Thr Gly Ala Val Leu Pro Ala Leu Ala Ala Ile Gly Glu Asp
                        565                 570                 575

        Met Thr Gln Pro Tyr Ile Thr Lys Ala Cys Asp Trp Leu Val Ala His
                    580                 585                 590

        Gln Gln Glu Asp Gly Gly Trp Gly Glu Ser Cys Ser Ser Tyr Met Glu
                595                 600                 605

        Ile Asp Ser Ile Gly Lys Gly Pro Thr Thr Pro Ser Gln Thr Ala Trp
            610                 615                 620

        Ala Leu Met Gly Leu Ile Ala Ala Asn Arg Pro Glu Asp Tyr Glu Ala
        625                 630                 635                 640

        Ile Ala Lys Gly Cys His Tyr Leu Ile Asp Arg Gln Glu Gln Asp Gly
                        645                 650                 655

        Ser Trp Lys Glu Glu Glu Phe Thr Gly Thr Gly Phe Pro Gly Tyr Gly
                    660                 665                 670

        Val Gly Gln Thr Ile Lys Leu Asp Asp Pro Ala Leu Ser Lys Arg Leu
                675                 680                 685

        Leu Gln Gly Ala Glu Leu Ser Arg Ala Phe Met Leu Arg Tyr Asp Phe
            690                 695                 700

        Tyr Arg Gln Phe Phe Pro Ile Met Ala Leu Ser Arg Ala Glu Arg Leu
        705                 710                 715                 720

        Ile Asp Leu Asn Asn
                        725
```

Das Plasmid pDHE-Zm-SHC-1 wurde in den Stamm *E. coli* TG10 *p*Agro4 *p*HSG575 [Takeshita et al., Gene 1987, 61:63-74; Tomoyasu et al., Mol Microbiol 2001, 40:397-413] transformiert. Die rekombianten *E. coli* erhalten die Bezeichnung *E. coli* LU15568.

**Beispiel 2: Bereitstellung rekombinanter Homofamesol-Zyklase aus *Z. mobilis***

Aus einer entsprechenden 2mL Vorkultur angeimpft, wurde *E. coli* LU15568 in 20mL LB-Amp/Spec/Cm (100μg/l Ampicillin; 100μg/l Spectinomycin; 20μg/l Chloramphenicol), 0,1mM IPTG, 0,5g/L Rhamnose in 100mL Erlenmeyerkolben (Schikanen) 16 h bei 37°C angezogen, bei 5000*g/10min zentrifugiert und bei 4°C gelagert. Proteinextrakt wurde hergestellt indem das Zellpellet in 15mL Aufschlusspuffer (0,2M Tris/HCl, 0,5M EDTA, pH 8.0), 375U Benzonase (z.B. Novagen, 25U/μL), 40μL PMSF (100mM, gelöst in *i*-PropOH), 0,8g Saccharose und ca. 0.5mg Lysozym suspendiert wurden. Den Ansatz wurde gemischt und 30min auf Eis inkubiert. Anschließend wurde die Mischung bei -20°C eingefroren.

Nach dem Auftauen des Ansatzes wurden mit Aqua dest. auf ca. 40mL aufgefüllt und nochmals für 30min auf Eis inkubiert.

Anschließend wurden die Zellen 3mal 3min mit Ultraschall aufgeschlossen (HTU-Soni 130, Fa. G. Heinemann, Schwäbisch-Hall, Amplitude 80%, 15" Puls / 15" Pause). Nach dem Aufschluss wurden die Zelltrümmer in 60min bei 4°C und 26900 * *g* abzentrifugiert. Der Überstand wurde verworfen und das Pellet in 100mL Solubilisationspuffer (50mM Tris/HCl, 10mM MgCl2x6H2O, 1% Triton X-100, pH 8.0) resuspendiert und ca. 5min gepottert. Anschließend wurde die Suspension für 30min auf Eis gehalten.

Der homogenisierte Extrakt wurde erneut für 1h bei 4°C und 26900 * *g* zentrifugiert und das Pellet verworfen. Der Extrakt wurde für die Enzymtests eingesetzt und kann mehrere Wochen ohne Aktivitätsverlust bei -20°C gelagert werden. Der Proteingehalt liegt im Bereich von 1 mg/mL.

**Beispiel 3: Aktivitätsbestimmung der rekombinanten Zyklase aus *E. coli* LU15568**

Mit der in Beispiel 2 beschriebenen Proteinpräparation wurde Homofarnesylsäure (1b, (3*E*,7*E*)-4,8,12-trimethyltrideca-3,7,11-triensäure)) inkubiert. Im Einzelnen wurden 0,0412g Homofarnesylsäure abgewogen (20mM im Ansatz; Reinheit 85,1% bestehend aus *Z,Z* 0,44%, *E,Z* 10,13%, *E,E* 74,93%), 2,913mL Wasser, 0,350mL Natriumcitrat Puffer (1M Natriumcitrat pH 5,4), 0,560mL $MgCl_2$ (0,5M Lösung) dazu pipettiert und unter Rühren bei 37°C 30Min temperiert. Die Reaktion wurde mit Zugabe von auf 37°C temperiertem Homogenat aus *E. coli* LU15568 (Proteingehalt 35mg/ml) gestartet. Der Reaktionsansatz wurde 24h bei pH 5,0 und 37°C bei max. Rührgeschwindigkeit auf einem Magnetrührer im Ölbad gerührt. pH Ausgleich während der Reaktion erfolgte mit 0,5M HCl. Nach einer 24 stündigen Inkubation wurden 0,500mL aus dem Ansatz mit 1,000mL *n*-Heptan / *n*-Propanol 3:2 30 Sekunden mittels Vortexen extrahiert. Der organische Überstand nach Phasentrennung wurde für die GC Analyse eingesetzt (vgl. Figur 1)

Mit der in unten näher beschriebenenAnalytik konnte ein Umsatz von 74,5% gesamt bzw. 82,7% aus dem *E,E* Isomer festgestellt werden.

Der Umsatz von Homofarnesylsäure (1b) zu Sclareolid (3) lässt sich mit folgendem GC-System bestimmen:

Säule: 10m Optima 1
Temperaturprofil:
0 min: 100°C
5°C/min auf 200°C
Nach 5 min:30°C/min auf 320°C danach konstant
Methodendauer: 30 min
Injektortemperatur: 280°C
Retentionszeiten (RT):
Homofarnesylsäure: Peak 1 bei 11,7 min, Peak 2 bei 12,1 min ;
Sclareolid: ca. 13,5 min

Mit authentischem Material (Sigma-, Kat. No.: 358002) wurde eine Eichreihe erstellt, anhand derer die Konzentration unbekannter Proben bestimmt wurde.

Auf die Offenbarung der hierin zitierten Druckschriften wird ausdrücklich Bezug genommen.

**Sequenzen:**

SEQ ID NO: 1- 326 Nukleinsäure/Aminosäuresequenzen verschiedener SHC Gene SEQ ID NO: 327-328 PCR Primer

Es folgt eine Auflistung erfindungsgemäß besonders brauchbarer SHC-Enzymsequenzen: Enzymsequenzen

>seq_ID 4
MNMASRFSLKKILRSGSDTQGTNVNTLIQSGTSDIVRQKPAPQEPADLSALKAMGNSLTHTLS-
SACEWLMKQQKPDGHWVGSVGSNASMEAEWCLALWFLGLEDHPLRPRLGKAL-
LEMQRPDGSWGTYYGAGSGDINATVESYAALRSLGYAEDDPAVSKAAA-
WIISKGGLKNVRVFTRYWLALIGEWPWEKTPNLPPEIIWFPDNFVFSIYNFAQWARATMM-
PLAILSARRPSRPLRPQDRLDALFPGGRANFDYELPTKEGRDVIADFFRLADKGLHWLQSS-
FLKRAPSREAAIKYVLEWIIWHQDADGGWGGIQPPWVYGLMALHGEGYQFHHPVMAKAL-
DALNDPGWRHDKGDASWIQATNSPVWDTMLSLMALHDANAEERFTPEMDKALD-
WLLSRQVRVKGDWSVKLPNTEPGGWAFEYANDRYPDTDDTAVALIAIASCRNRPEWQAKG-
VEEAIGRGVRWLVAMQSSCGGWGAFDKDNNKSILAKIPFCDFGEALDPPSVDVTAHVLEAF-
GLLGLPRDLPCIQRGLAYIRKEQDPTGPWFGRWGVNYLYGTGAVLPALAALGEDMTQPYIS-
KACDWLINCQQENGGWGESCASYMEVSSIGHGATTPSQTAWALMGLIAANRPQDYEAI-
AKGCRYLIDLQEEDGSWNEEEFTGTGFPGYGVGQTIKLDDPAISKRLMQGAELSRAF-
MLRYDLYRQLFPIIALSRASRLIKLGN

>seq_ID 2
MGIDRMNSLSRLLMKKIFGAEKTSYKPASDTIIGTDTLKRPNRRPEPTAKVDKTI-FKTMGNSLNNTLVSACDWLIGQQKPDGHWVGAVESNASMEAEWCLALWFLGLEDH-PLRPRLGNALLEMQREDGSWGVYFGAGNGDINATVEAYAALRSLGYSADNPVLKKAAA-WIAEKGGLKNIRVFTRYWLALIGEWPWEKTPNLPPEIIWFPDNFVFSIYNFAQWA-RATMVPIAILSARRPSRPLRPQDRLDELFPEGRARFDYELPKKEGIDLWSQFFRTT-DRGLHWVQSNLLKRNSLREAAIRHVLEWIIRHQDADGGWGGIQPPWVYGLMALH-GEGYQLYHPVMAKALSALDDPGWRHDRGESSWIQATNSPVWDTMLALMALKDAKAEDRFT-PEMDKAADWLLARQVKVKGDWSIKLPDVEPGGWAFEYANDRYPDTDDTAVALIALSSYRD-KEEWQKKGVEDAITRGVNWLIAMQSECGGWGAFDKDNNRSILSKIPFCDFGESI-DPPSVDVTAHVLEAFGTLGLSRDMPVIQKAIDYVRSEQEAEGAWFGRWGVNYIYGTGAVLPA-LAAIGEDMTQPYITKACDWLVAHQQEDGGWGESCSSYMEIDSIGKGPTTPSQTAWALM-GLIAANRPEDYEAIAKGCHYLIDRQEQDGSWKEEEFTGTGFPGYGVGQTIKLD-DPALSKRLLQGAELSRAFMLRYDFYRQFFPIMALSRAERLIDLNN

>seq_ID 5
MTVTSSASARATRDPGNYQTALQSTVRAAADWLI-ANQKPDGHWVGRAESNACMEAQWCLALWFMGLEDHPLRKRLGQSLLDSQRPD-GAWQVYFGAPNGDINATVEAYAALRSLGFRDDEPAVRRAREWIEAKGGLRNIRVFTRYWLA-LIGEWPWEKTPNIPPEVIWFPLWFPFSIYNFAQWARATLMPIAVLSARRPSRPLPPEN-RLDALFPHGRKAFDYELPVKAGAGGWDRFFRGADKVLHKLQNLGNRLNLGLFRPAATSRV-LEWMIRHQDFDGAWGGIQPPWIYGLMALYAEGYPLNHPVLAKGLDALND-PGWRVDVGDATYIQATNSPVWDTILTLLAFDDAGVLGDYPEAVD-KAVDWVLQRQVRVPGDWSMKLPHVKPGGWAFEYANNYYPDTDDTAVALIAL-APLRHDPKWKAKGIDEAIQLGVDWLIGMQSQGGGWGAFDKDNNQKILTKIPFCDYGEALD-PPSVDVTAHIIEAFGKLGISRNHPSMVQALDYIRREQEPSGPWFGRWGVNYVYGTGAVLPA-LAAIGEDMTQPYIGRACDWLVAHQQADGGWGESCASYMDVSAVGRGTTTASQTAWAL-MALLAANRPQDKDAIERGCMWLVERQSAGTWDEPEFTGTGFPGYGVGQTIKLND-PALSQRLMQGPELSRAFMLRYGMYRHYFPLMALGRALRPQSHS

>seq_ID 6
MTVSTSSAFHHSSLSDDVEPIIQKATRALLEKQHQDGHWVFELEADATIPAEYILLKHYLGE-PEDLEIEAKIGRYLRRIQGEHGGWSLFYGGDLDLSATVKAYFALKMIGDSPDAPHML-RARNEILARGGAMRANVFTRIQLALFGAMSWEHVPQMPVELMLMPEWFPVHINKMAYWART-VLVPLLVLQALKPVARNRRGILVDELFVPDVLPTLQESGDPIWRRFFSALDKVLHK-VEPYWPKNMRAKAIHSCVHFVTERLNGEDGLGAIYPAIANSVMMYDALGYPENHPERAIAR-RAVEKLMVLDGTEDQGDKEVYCQPCLSPIWDTALVAHAMLEVGGDEAEKSAISALS-WLKPQQILDVKGDWAWRRPDLRPGGWAFQYRNDYYPDVDDTAVVTMAM-DRAAKLSDLHDDFEESKARAMEWTIGMQSDNGGWGAFDANNSYTYLNNIPFADHGALLD-PPTVDVSARCVSMMAQAGISITDPKMKAAVDYLLKEQEEDGSWFGRWGVNYIYGTWSAL-CALNVAALPHDHLAIQKAVAWLKNIQNEDGGWGENCDSYALDYSGYEPMDSTASQTAWALL-GLMAVGEANSEAVTKGINWLAQNQDEEGLWKEDYYSGGGFPRVFYLRYHGYSKYFPL-WALARYRNLKKANQPIVHYGM

## Patentansprüche

1. Verfahren zur biokatalytischen Herstellung einer Verbindung der allgemeinen Formel II

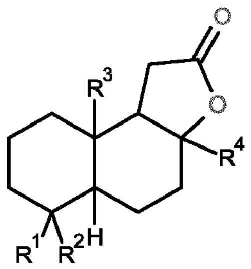

(II)

worin $R^1$, $R^2$, $R^3$ und $R^4$ unabhängig voneinander für H oder $C_1$-$C_4$-Alkyl stehen,
in stereoisomerenreiner Form oder als Gemisch von Stereoisomeren,
wobei man die Verbindung mit einem Protein in Kontakt bringt, das zur Zyklisierung einer mehrfach ungesättigten Carbonsäure, insbesondere von Homofarnesylsäure, befähigt ist.

2. Verfahren nach Anspruch 1, wobei man die Verbindung der Formel I mit einem Protein in Kontakt bringt, das die enzymatische Aktivität einer Squalen-Hopen-Zyklase (SHC) aufweist.

3. Verfahren nach Anspruch 1 oder 2 wobei man als Substrat eine mehrfach ungesättigte Carbonsäure der allgemeinen Formel I,

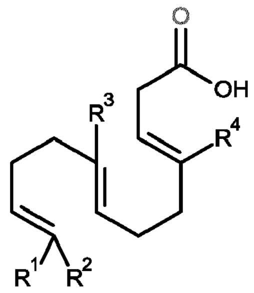

(I)

worin $R^1$, $R^2$, $R^3$ und $R^4$ die oben angegebenen Bedeutungen besitzen, einsetzt, insbesondere in im wesentlichen stereoisiomerenreiner Form.

**4.** Verfahren nach einem der vorherigen Ansprüche, wobei man Homofarnesylsäure der Formel Ia

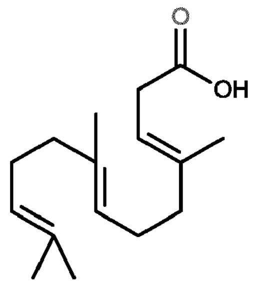

(Ia)

als Edukt einsetzt, insbesondere in im wesentlichen stereoisiomerenreiner Form, vorzugsweise in einem Anteil der (3E,7E)-Homofarnesylsaüre von wenigsten 70 mol%, besonders bevorzugt wenigsten 75, 80 oder 85 mol%, am meisten bevorzugt 90, 95 oder 99 mol%, bezogen auf die Gesamtmenge an enthaltenen Homofarnesylsäure-Isomeren.

**5.** Verfahren nach Anspruch 4, wobei man Sclareolid der Formel IIa

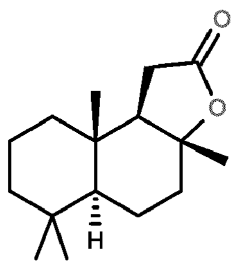

(IIa)

in stereoisomerenreiner Form oder als Gemisch von Stereoisomeren erhält.

**6.** Verfahren nach einem der vorherigen Ansprüche, wobei die SHC ausgewählt ist unter

a) Proteinen, umfassend ein Polypeptid mit einer Aminosäuresequenz gemäß SEQ ID NO: 2
b) durch Deletion, Insertion, Substitution, Addition, Inversion oder einer Kombination von Proteinen gemäß a) abgeleiteten Proteinen, umfassend ein Polypeptid mit einer Sequenzidentität von wenigsten 45% zur Aminosäuresequenz gemäß SEQ ID NO: 2; und

c) zu a) oder b) funktional äquivalenten Proteinen, welche die Zyklisierung von Homofarnesylsäure zu Sclareolid katalysieren.

**7.** Verfahren nach einem der vorhergehenden Ansprüche, wobei man die biokatalytische Umsetzung

a) bei einem pH Wert des Reaktionsmediums im Bereich von etwa 4 bis 5,8, insbesondere 4,5 bis 5,5; und unter wenigstens einer der folgenden weiteren Bedingungen durchführt:

b) bei einer Substratkonzentration von mindesten 15 mM, insbesondere 15 bis 30 mM;

c) bei einer Enzymkonzentration von wenigstens 5mg/ml;

d) bei einer Reaktionstemperatur im Bereich von 32 bis 40 °C, insbesondere 35 bis 38°C;

e) in einem Natrium-Citrat-Puffer enthaltend 1 bis 20 mM $MgCl_2$; und/oder

f) bei einer Pufferkonzentration von etwa 10 bis 100 mM

**8.** Verfahren nach einem der vorherigen Ansprüche, wobei die enzymatische Zyklase-Aktivität, insbesondere dir Aktivität der SHC, in einer Form vorliegt, ausgewählt unter:

a) einer freien, gegebenenfalls teilweise oder vollständig gereinigten natürlichen oder rekombinant hergestellten Zyklase,

b) Zyklase gemäß a) in immobilisierter Form;

c) ganzen Zellen, enthaltend mindestens eine Zyklase;

d) Zelllysaten oder Zellhomogenisaten von Zellen gemäß c).

**9.** Verfahren nach einem der vorhergehenden Ansprüche, wobei die Umsetzung in einphasigen wässrigen Systemen oder in zweiphasigen wässrig-organischen oder festflüssigem Systemen erfolgt.

**10.** Verfahren nach einem der vorhergehenden Ansprüche, wobei die Umsetzung bei einer Temperatur im Bereich von 37°C und einem pH-Wert im Bereich von5 bis 5,2 erfolgt.

**11.** Verfahren nach einem der vorhergehenden Ansprüche, wobei die SHC aus einem Mikroorganismus ausgewählt unter *Methylococcus capsalatus, Rhodopseudomonas palustris, Bradyrhizobium japonicum, Frankia spec., Streptomyces coelicolor* und insbesondere *Zymomonas mobilis* isoliert wird.

**12.** Verfahren nach einem der vorhergehenden Ansprüche, wobei die SHC aus einem die SHC überexprimierenden Mikroorganismus isoliert wird, welcher ausgewählt ist unter Bakterien der Gattung *Escherichia, Corynebacterium, Ralstonia, Clostridium, Pseudomonas, Bacillus, Zymomonas, Rhodobacter, Streptomyces, Burkholderia, Lactobacillus* und *Lactococcus,* insbesondere *Escherichia.*

**13.** Verfahren nach einem der vorhergehenden Ansprüche, wobei die SHC aus transgenen die SHC überexprimierenden Bakterien der Spezies *Escherichia coli, Pseudomonas putida, Burkholderia glumae, Streptomyces lividans, Streptomyces coelicolor* und *Zymomonas mobilis,* insbesondere *E. coli,* isoliert wird.

**14.** Verfahren zur Herstellung von 3a,6,6,9a-Tetramethyl-dodecahydronaphto[2,1-b]furan (Ambroxid), wobei man

a) Homofarnesylsäure nach einem Verfahren gemäß einem der Ansprüche 1 bis 13 zu Sclareolid umsetzt;

b) das Produkt aus Stufe a) chemisch zu Ambroxdiol reduziert und

c) Ambroxidol aus Stufe b) chemisch zu Ambroxid cyclisiert.

**15.** Verfahren nach Anspruch 14, wobei Ambroxid das (-)- Ambroxid ((3aR,5aS,9aS,9bR)-3a,6,6,9a-Tetramethyl-dodecahydronaphto[2,1-b]furan [CAS 6790-58-5]) ist.

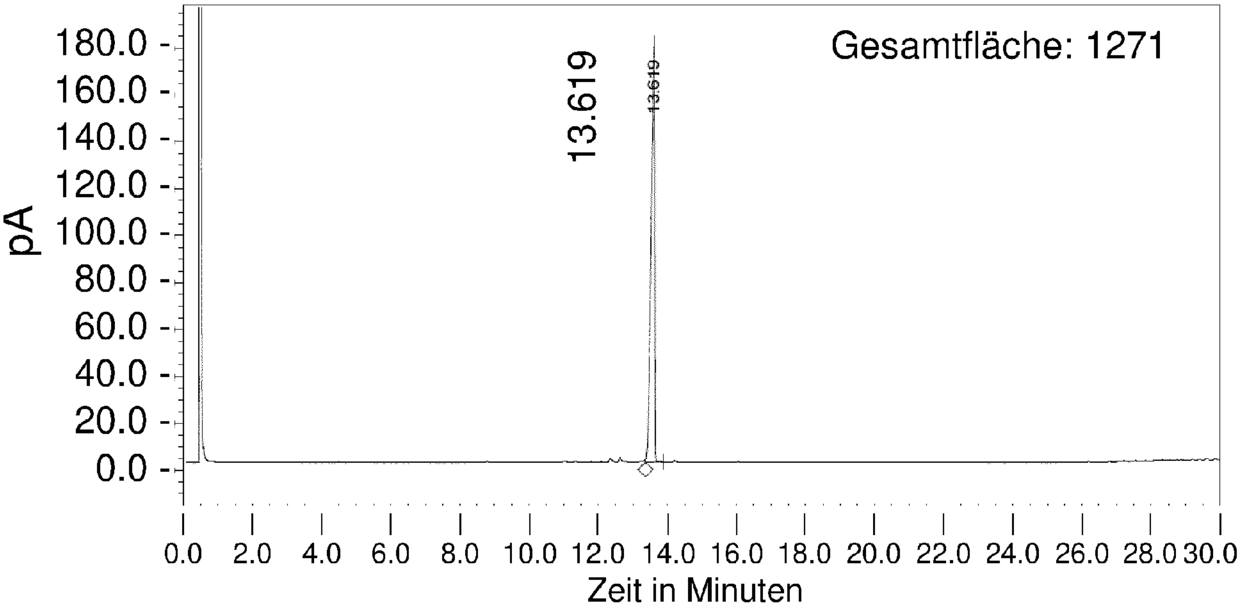
Gesamtfläche: 1271
13.619
pA
180.0
160.0
140.0
120.0
100.0
80.0
60.0
40.0
20.0
0.0
0.0 2.0 4.0 6.0 8.0 10.0 12.0 14.0 16.0 18.0 20.0 22.0 24.0 26.0 28.0 30.0
Zeit in Minuten

Fig. 1A

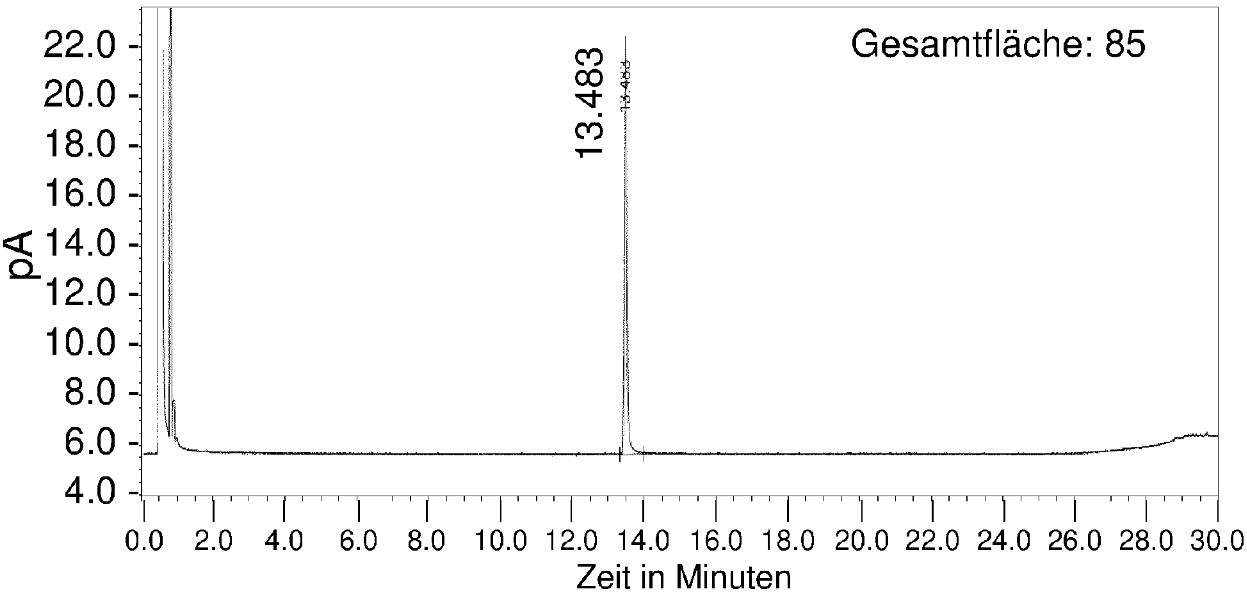
Gesamtfläche: 85
13.483
pA
22.0
20.0
18.0
16.0
14.0
12.0
10.0
8.0
6.0
4.0
0.0 2.0 4.0 6.0 8.0 10.0 12.0 14.0 16.0 18.0 20.0 22.0 24.0 26.0 28.0 30.0
Zeit in Minuten

Fig. 1B

Europäisches Patentamt
European Patent Office
Office européen des brevets

# EUROPÄISCHER RECHERCHENBERICHT

Nummer der Anmeldung
EP 20 18 8856

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br><br><br><br><br><br><br>A | SEITZ, M. ET AL.: "Synthesis of Heterocyclic Terpenoids by Promiscuous Squalene-Hopene Cyclases", CHEMBIOCHEM, Bd. 14, Nr. 4, 18. Februar 2013 (2013-02-18), Seiten 436-439, XP002760197,<br>* das ganze Dokument *<br>* siehe insbesondere: *<br>* Seite 436, Spalte 2, Zeilen 26-36 *<br>* Seite 437, Spalte 1, Zeile 8 - Spalte 2, Zeile 7; Tabelle 1; Verbindungen 3, 3a *<br>* Seite 438 *<br>* Schema 2 *<br>* Seite 439, Spalte 1, Zeilen 3-9 * | 1-13<br><br><br><br><br><br><br>14,15 | INV.<br>C12P17/04 |
| X<br><br><br><br><br><br><br><br><br><br><br><br><br><br><br>A | -& SEITZ, M. ET AL.: "Supporting Information to: Synthesis of Heterocyclic Terpenoids by Promiscuous Squalene-Hopene Cyclases", CHEMBIOCHEM, Bd. 14, Nr. 4 18. Februar 2013 (2013-02-18), Seiten 1-31, XP002760210, Gefunden im Internet: URL:http://onlinelibrary.wiley.com/store/10.1002/cbic.201300018/asset/supinfo/cbic_201300018_sm_miscellaneous_information.pdf?v=1&s=2b12f2a3197e5694b6242f266d423c8883ac74e7 [gefunden am 2016-07-25]<br>* Seite 2, Absatz 1.2-1.3 *<br>* Seiten 2-3, Absatz 1.5 *<br>* Seite 6, Absatz 2.2 *<br>* Seite 15; Abbildung 13 *<br>* Seite 16; Abbildung 14 *<br>-----<br>-/-- | 1-13<br><br><br><br><br><br><br><br><br><br><br><br><br><br><br>14,15 | RECHERCHIERTE SACHGEBIETE (IPC)<br>C12P |

Der vorliegende Recherchenbericht wurde für alle Patentansprüche erstellt

1

| Recherchenort | Abschlußdatum der Recherche | Prüfer |
|---|---|---|
| Berlin | 25. März 2021 | Fuchs, Ulrike |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 20 18 8856

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X<br><br>A | US 2012/237991 A1 (BREUER, M. ET AL.)<br>20. September 2012 (2012-09-20)<br>* Seite 6, Spalte 1, Absatz 88 - Spalte 2, Absatz 114 *<br>* Seite 7, Spalte 1, Absatz 129 *<br>* Seite 8, Spalte 2 - Seite 9, Spalte 1; Sequenzen 2, 6, 16 *<br>* Seiten 25-26, Absatz 352 *<br>* Seite 27; Tabelle A; Verbindung Homofarnesylic acid *<br>* Seiten 122-125; Ansprüche 27, 47, 49 *<br>* Sequenzen 2, 6, 16 *<br>----- | 1-13<br><br>14,15 | |
| X,D<br><br>A | WO 2010/139719 A2 (BASF SE)<br>9. Dezember 2010 (2010-12-09)<br>* Seite 1, Zeilen 9-18 *<br>----- | 14,15<br><br>1-13 | |
| | | | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| Berlin | 25. März 2021 | Fuchs, Ulrike |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 20 18 8856

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-03-2021

| Im Recherchenbericht angeführtes Patentdokument | Datum der Veröffentlichung | Mitglied(er) der Patentfamilie | Datum der Veröffentlichung |
|---|---|---|---|
| US 2012237991 A1 | 20-09-2012 | US 2012237991 A1 | 20-09-2012 |
| | | US 2015104851 A1 | 16-04-2015 |
| | | US 2016340666 A1 | 24-11-2016 |
| | | US 2019119665 A1 | 25-04-2019 |
| WO 2010139719 A2 | 09-12-2010 | CN 102449158 A | 09-05-2012 |
| | | EP 2438182 A2 | 11-04-2012 |
| | | EP 3404108 A1 | 21-11-2018 |
| | | ES 2703770 T3 | 12-03-2019 |
| | | JP 5735493 B2 | 17-06-2015 |
| | | JP 2012528578 A | 15-11-2012 |
| | | US 2012135477 A1 | 31-05-2012 |
| | | WO 2010139719 A2 | 09-12-2010 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 204009 A
- US 513270 A
- WO 2012066059 A
- EP 2010057696 W
- WO 2005108590 A
- WO 2006094945 A
- EP 1149849 A
- EP 1069183 A
- DE OS100193773 A


### In der Beschreibung aufgeführte Nicht-Patentliteratur


- **STOLL et al.** *Helv Chim Acta,* 1950, vol. 33, 1251
- **MOOKHERJEE et al.** *Perfumer and Flavourist,* 1990, vol. 15, 27
- **LUCIUS et al.** *Chem Ber,* 1960, vol. 93, 2663
- **BÜCHI et al.** *Helv Chim Acta,* 1989, vol. 72, 996
- **NEUMANN et al.** *Biol Chem Hoppe Seyler,* 1986, vol. 367, 723
- **SEITZ, M. et al.** *ChemBioChem,* 2013, vol. 14, 436-439
- *Enzymcode gemäß Eur. J. Biochem.,* 1999, vol. 264, 610-650
- **OCHS D. et al.** *J Bacteriol,* 1992, vol. 174, 298
- **PEARSON ; LIPMAN.** *Proc. Natl. Acad, Sci.,* 1988, vol. 85 (8), 2444-2448
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **HIGGINS DG ; SHARP PM.** Fast and sensitive multiple sequence alignments on a microcomputer. *Comput Appl. Biosci.,* April 1989, vol. 5 (2), 151-1
- **CHENNA ; RAMU ; SUGAWARA ; HIDEAKI ; KOIKE ; TADASHI ; LOPEZ ; RODRIGO ; GIBSON ; TOBY J.** Multiple sequence alignment with the Clustal series of programs. *Nucleic Acids Res,* 2003, vol. 31 (13), 3497-500
- Phosphoamiditmethode. Wiley Press, 896-897
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology. IRL Press at Oxford University Press, 1991
- **SAMBROOK, J. ; FRITSCH, E.F. ; MANIATIS, T.** Molecular Cloning (A Laboratory Manual). Cold Spring Harbor Laboratory Press, 1989, 9.31-9.57
- Current Protocols in Molecular Biology. John Wiley & Sons, 1989, 6.3.1-6.3.6
- **SAMBROOK ; RUSSELL.** Molecular Cloning. Cold Spring Harbor Laboratory Press, 2001
- Nukleotide eines Gens ausgetauscht werden. Humana Press, 1996
- **KEGLER-EBO DM ; DOCKTOR CM ; DIMAIO D.** *Nucleic Acids Res,* 1994, vol. 22, 1593
- **BARETTINO D ; FEIGENBUTZ M ; VALCÁREL R ; STUNNENBERG HG.** *Nucleic Acids Res,* 1994, vol. 22, 541
- **BARIK S.** *Mol Biotechnol,* 1995, vol. 3, 1
- **ECKERT KA ; KUNKEL TA.** *Nucleic Acids Res,* 1990, vol. 18, 3739
- **SCHENK et al.** *Biospektrum,* 2006, vol. 3, 277-279
- An efficient random mutagenesis technique using an E.coli mutator strain. **GREENER A ; CALLAHAN M ; JERPSETH B.** In vitro mutagenesis protocols. Humana Press, 1996
- **STEMMER WPC.** *Nature,* 1994, vol. 370, 389
- **STEMMER WPC.** *Proc Natl Acad Sci USA,* 1994, vol. 91, 10747
- **REETZ MT ; JAEGER K-E.** *Topics Curr Chem,* 1999, vol. 200, 31

- Methods for optimizing industrial enzymes by directed evolution. **ZHAO H ; MOORE JC ; VOLKOV AA ; ARNOLD FH.** Manual of industrial microbiology and biotechnology. American Society for Microbiology, 1999
- **GOEDDEL.** Gene Expression Technology: Methods in Enzymology. Academic Press, 1990, vol. 185
- Buch Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc, 1987
- Cloning Vectors. Elsevier, 1985
- Current Protocols in Molecular Biology. Wiley Interscience, 1997
- **SAMBROOK et al.** Molecular Cloning: A Laboratory Manual. Cold Spring Harbor Laboratory, 1989
- Bioprozeßtechnik 1. Einführung in die Bioverfahrenstechnik. Gustav Fischer Verlag, 1991
- Bioreaktoren und periphere Einrichtungen. Vieweg Verlag, 1994
- Handbuch ''Manual of Methods für General Bacteriology. American Society für Bacteriology, 1981
- Applied Microbiol. Physiology, A Practical Approach. IRL Press, 1997, 53-73
- **COOPER, T. G.** Biochemische Arbeitsmethoden. Verlag Walter de Gruyter
- **SCOPES, R.** Protein Purification. Springer Verlag
- **HARLOW, E. ; LANE, D.** Antibodies: A Laboratory Manual. Cold Spring Harbor (N.Y.) Press, 1988
- **J. LALONDE ; A. MARGOLIN.** *Immobilization of Enzymes*
- **K. DRAUZ ; H. WALDMANN.** Enzyme Catalysis in Organic Synthesis. Wiley-VCH, 2002, vol. III, 991-1032
- Biotechology. VCH, vol. 3
- **SAMBROOK, J. ; FRITSCH, E.F. ; MANIATIS, T.** Molecular cloning: A Laboratory Manual. Cold Spring Harbor Laboratory, 1989
- **TAKESHITA et al.** *Gene,* 1987, vol. 61, 63-74
- **TOMOYASU et al.** *Mol Microbiol,* 2001, vol. 40, 397-413